# EUROPEAN PATENT SPECIFICATION

(11) **EP 0 454 367 B1**
(45) Date of publication and mention of the grant of the patent: **10.11.1999**
(21) Application number: 91303507.7
(22) Date of filing: 19.04.1991
(51) Int. Cl.: C12N 15/11, C07K 14/60

(54) **Growth hormone releasing factor analogs**
Wachstumshormon-Releasingfaktor-Analoge
Analogues du facteur libérant l'hormone de croissance

(30) Priority: 24.04.1990 US 513730; 24.09.1990 US 587323
(43) Date of publication of application: 30.10.1991
(73) Proprietor: ELI LILLY AND COMPANY, Indianapolis, Indiana 46285 (US)
(72) Inventor: Brooke, Gerald Stephen, Indianapolis, IN 46220 (US); Dimarchi, Richard Dennis, Carmel, IN 46032 (US); Heiman, Mark Louis, Indianapolis, IN 46250 (US); Hsiung, Hansen Maxwell, Indianapolis, IN 46032 (US); Smiley, David Lee, Greenfield, IN 46140 (US); Smith, Dennis Paul, Indianapolis, IN 46254 (US)
(74) Representative: Hudson, Christopher Mark

(56) References cited:
- EP-A- 0 220 958
- EP-A- 0 264 750
- EP-A- 0 314 866
- EP-A- 0 361 956
- JOURNAL OF BIOTECHNOLOGY, vol. 12, no. 3/4, December 1989, Amsterdam (NL); R.J. KIRSCHNER et al., pp. 247-259

## Description

Growth hormone releasing factor (GRF) was originally isolated as a 44 amino acid peptide from a human pancreatic tumor. Guillemin et al., Science 218:585 (1982). GRF is a useful peptide because it stimulates the release of endogenous growth hormone. Because of its small size, GRF has been difficult to produce in large quantities by recombinant methods.

Previous attempts at expressing GRF in E. coli were unsatisfactory in two aspects. Direct expression resulted in the production of very low levels of protein. U.S. Patent 4,728,609. A high level of expression has only been achieved by fusion to another polypeptide, which required cleavage of the fusion polypeptide to yield active GRF. Villa et al., Eur. J. Biochem. 171:137 (1988); European Patent Application No. 0199018.

European Patent Appliction No. 0 220 958 discloses certain modified peptides of the form H-X-Pro-Peptide and in particular one in which the peptide is GRF and X is a methionine residue. European Patent Appliction Nos. 0 314 865 and 0 264 750 diclose growth hormone-releasing peptides that can be amidated at the C-terminus.

The present invention provides polypeptides that can be expressed at high levels in E. coli, yet require no cleavage to be active. Also provided are polypeptide compounds with GRF activity that are extended at their amino termini by one, two or three amino acids. Also provided are des-amino tyrosine derivatives of GRF. The compounds are produced by any means of synthesis, including solid phase peptide synthesis, solution peptide synthesis, enzymatic synthesis, semi-synthetic methods, and recombinant DNA methods.

The present invention provides polypeptide compounds with growth hormone releasing factor (GRF) activity that are expressed at high levels in E. coli. The polypeptide compounds also are produced by other means of peptide synthesis, including solid phase synthetic methods. Also provided are DNA compounds encoding these polypeptides. The polypeptide compounds which can be expressed at high levels in E. coli are represented by the formula 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 wherein A is Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr or Met-Ser-Pro-Tyr; R is Asn or Ser; B is Arg or Lys; C is Gly, Ala, Leu, Val, Ile, or Thr; D is Arg or Lys; E is Met, Val, Leu, or Ser; X is a chain of amino acid residues of sufficient length such that high-level expression in E. coli is achieved; none, one, or more than one of the amino acids having a free amino group is chemically modified; and the pharmaceutically acceptable non-toxic acid addition salts or carboxylic acid salts thereof.

For purposes of the present invention, "chemically modified" is defined as the derivatization of an amino acid's free amino group with an alkyl or hydroxyalkyl group. None, one or more than one of the free amino groups may be chemically modified. The extent of modification is controlled by length of reaction or amount of reagent. It is preferred that all amino acids having a free amino group are chemically modified. Especially preferred is a compound which only has one free amino group at the amino terminus that can be chemically modified. Such a compound has no lysines or methionines, other than at the N-terminus. Lysine and/or methionine-containing compounds are converted to compounds with a free amino group only at the N-termini by replacing the lysines with arginines and the methionines with leucines.

Preferred choices for A are Met-Pro-Tyr and Met-Ala-Tyr; an especially preferred choice is Met-Ala-Tyr. Preferred amino acids at C and E are Thr and Leu respectively. While X can be any chain of amino acids of sufficient length to achieve high-level expression of the compound in E. coli, preferred chain lengths are about 31-100 amino acids. More preferred are lengths of about 40-80 amino acids; especially preferred are lengths of about 45-55 amino acids. An especially preferred choice of amino acids for X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly. This amino acid sequence represents the C-terminal 48 amino acids of the human GRF precursor with all of the methionines replaced by leucines. The lysines in the above sequence are replaced by arginines to yield another preferred choice for X: Gln-Gln-Gly-Glu-Arg-Asn-Gln-Glu-Gln-Gly-Ala-Arg-Val-Arg-Leu-Gly-Arg-Gln-Val-Asp-Ser-Leu-Trp-Ala-Glu-Gln-Arg-Gln-Leu-Glu-Leu-Glu-Ser-Ile-Leu-Val-Ala-Leu-Leu-Gln-Arg-His-Ser-Arg-Asn-Ser-Gln-Gly. This sequence is preferred because the resulting compound has only one free amino group if B and D are arginines, thus facilitating the generation of homogeneous chemically modified compound.

Other preferred amino acid sequences at X include peptides from the foot and mouth disease virus surface protein and an amino acid sequence which constitutes GRF, thus creating a GRF dimer. Suitable sequences at X include Gln-Gln-Gly-Glu-Arg-Asn-Gln-Glu-Gln-Gly-Ala-Lys-Val-Arg-Leu-Tyr-Ala-Asp-Ala-Ile-Phe-Thr-Asn-Ser-Tyr-Arg-Lys-Val-Leu-Thr-Gln-Leu-Ser-Ala-Arg-Lys-Leu-Gln-Asp-Ile-Leu-Asn-Arg-Gln-Gln-Gly-Glu-Arg-Asn-Gln-Glu-Gln-Gly-Ala-Lys-Val-Arg-Leu and Gln-Gln-Gly-Glu-Arg-Asn-Glu-Glu-Gln-Gly-Ala-Lys-Val-Arg-Leu-Gly-Arg-Gln-Val-Asp-Ser-Met-Trp-Ala-Glu-Gln-Lys-Gln-Met-Glu-Leu-Glu-Ser-Ile-Leu-Val-Ala-Leu-Leu-Gln-Lys-His-Ser-Arg-Asn-Ser-Gln-Gly.

Other polypeptide compounds of the invention are of the formula 
A-Ala-Asp-Ala-Ile-Phe-Thr-Asn-R-Tyr-Arg-B-Val-
5 10
Leu-C-Gln-Leu-Ser-Ala-Arg-D-Leu-Leu-Gln-Asp-Ile-E-
15 20 25
Asn-Arg-X;
 wherein A is Met, desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, or Met-Ser-Pro-Tyr; R is Asn or Ser; B is Arg or Lys; C
is Gly, Ala, Leu, Val, Ile, or Thr; D is Arg or Lys; E is Met, Val, Leu or Ser; X is an amino acid chain with a length of greater than 30 amino acids; none, one, or more than one of the amino acids having a free amino group is chemically modified; and the pharmaceutically acceptable non-toxic acid addition salts or carboxylic acid salts thereof.

Related polypeptide compounds for comparative purpose are of the formula 
A-Ala-Asp-Ala-Ile-Phe-Thr-Asn-Ser-Tyr-Arg-B-Val-
5 10
Leu-C-Gln-Leu-Ser-Ala-Arg-D-Leu-Leu-Gln-Asp-Ile-E-Asn-
15 20 25
Arg-X;
 wherein A is Met, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, and Met-Ser-Pro-Tyr; B is Arg or Lys; C is Gly, Ala, Leu, Val, Ile, or Thr; D is Arg or Lys; E is Met, Val, Leu, or Ser; X is NH₂ or a chain of amino acids with a length of 1-30; none, one, or more than one of the amino acids having a free amino group is chemically modified; and the pharmaceutically acceptable non-toxic acid addition salts and the carboxylic acid salts thereof.

The polypeptide compounds of the invention differ from natural bGRF in several respects. The amino acid sequence of natural bGRF is 
Tyr-Ala-Asp-Ala-Ile-Phe-Thr-Asn-Ser-Tyr-Arg-Lys-Val-
5 10

Leu-Gly-Gln-Leu-Ser-Ala-Arg-Lys-Leu-Leu-Gln-Asp-Ile-
15 20 25

Met-Asn-Arg-Gln-Gln-Gly-Glu-Arg-Asn-Gln-Glu-Gln-Gly-
30 35

Ala-Lys-Val-Arg-Leu.
40
 In contrast, the present invention provides GRF analogs which differ from natural bGRF at the amino and/or carboxy terminii. Also included are analogs with the glycine at position 15 replaced by threonine.

The amino terminus of natural GRF is the tyrosine at position 1. The polypeptide compounds of the invention, except where A is Met, are extended at the N-terminus. This extension is designated by the variable A. When produced in E. coli, the compounds of the invention wherein A begins with methionine followed by alanine, glycine, proline, or serine undergo natural processing which releases the initiator methionine. This process yields the compounds of the invention where A begins with alanine, glycine, proline, or serine. The compound wherein A is Met yields a poorly active compound after removal of the methionine but the resulting compound is useful as a substrate for the creation of compounds with position 1 substitutions, such as the compound wherein the tyrosine of position 1 is substituted with desNH₂-tyrosine. The compounds of the invention that do not begin with methionine are produced via synthetic methods, including solid phase peptide synthesis. Alternatively, the compounds wherein A is Gln-Tyr, His-Tyr, Phe-Tyr, Arg-Ala-Tyr, Asp-Ala-Tyr, Phe-Ala-Tyr, and E is valine, leucine or serine are produced by recombinant techniques followed by cleavage of an initiator methionine with cyanogen bromide. Cyanogen bromide cleavage can only be utilized when the amino acids denoted by X do not include methionine. Skilled artisans will recognize that other methods of generating the compounds of the invention can be employed.

Some polypeptides of the invention have amino acids at the carboxy-terminus which are defined by X. It is known that the active portion of native GRF resides in the N-terminal 29 amino acids. Rivier et al., Nature 300:276 (1982). The polypeptide compounds of the invention where X is a chain of amino acids of length greater than 30 result from the discovery that the compound can be extended to a length such that high-level expression in E. coli is achieved, yet GRF activity is retained.

In the context of this invention, it is believed that a minimum total length of about 60 amino acids is required to achieve high yields of GRF in E. coli. The C-terminal extensions defined by X (when X is a chain of amino acids of length greater than 30) are based on the discovery that any chain of amino acids can be used to extend the chain and retain GRF activity. However, a bovine GRF compound with the C-terminal 33 amino acids of the human GRF precursor (the preferred polypeptide compound of the invention) is most active in cattle and sheep. The compound has some activity in pigs, cats and chickens.

The present invention includes the chemically modified forms of the polypeptide compounds of the invention. Methods of alkylating peptides are well known in the art. Acharya and Manjula, Biochem. 26:3524 (1987); Brown and Greenberg, Anal. Letters 17:1429 (1984). The alkylated derivatives are substituted at their free amino groups by a C₁-C₆ straight or branched chain alkyl group substituted with zero, one or more than one hydroxyl group. Preferred groups are C₁-C₃ straight chain hydroxyalkyl groups. Especially preferred substitutions are methyl, 2-hydroxyethyl and 2,3-dihydroxypropyl. Most preferred is 2,3-dihydroxypropyl.

A preferred polypeptide compound is 
Ala-Tyr-Ala-Asp-Ala-Ile-Phe-Thr-Asn-Ser-Tyr-Arg-Lys-
0                    5                    10
Val-Leu-Thr-Gln-Leu-Ser-Ala-Arg-Lys-Leu-Leu-Gln-Asp-
        15                  20                      25
Ile-Leu-Asn-Arg-Gln-Gln-Gly-Glu-Arg-Asn-Gln-Glu-Gln-
                30                  35
Gly-Ala-Lys-Val-Arg-Leu-Gly-Arg-Gln-Val-Asp-Ser-Leu-
    40                  45                      50
Trp-Ala-Glu-Gln-Lys-Gln-Leu-Glu-Leu-Glu-Ser-Ile-Leu-
            55                  60
Val-Ala-Leu-Leu-Gln-Lys-His-Ser-Arg-Asn-Ser-Gln-Gly.
65                  70                  75
 Especially preferred is the derivative of the above compound wherein the free amino groups of the alanine at position 0 and the lysines at positions 12, 21, 41, 56 and 70 are substituted with 2,3-dihydroxypropyl. This compound was found to have greater growth hormone releasing factor activity than the native molecule in vivo. Another preferred compound is derived from the above compound by replacing all five lysines with arginines, yielding 
Ala-Tyr-Ala-Asp-Ala-Ile-Phe-Thr-Asn-Ser-Tyr-Arg-Arg-
0                   5                   10
Val-Leu-Thr-Gln-Leu-Ser-Ala-Arg-Arg-Leu-Leu-Gln-Asp-
    15                  20                      25
Ile-Leu-Asn-Arg-Gln-Gln-Gly-Glu-Arg-Asn-Gln-Glu-Gln-
            30                  35
Gly-Ala-Arg-Val-Arg-Leu-Gly-Arg-Gln-Val-Asp-Ser-Leu-
    40                  45                  50
Trp-Ala-Glu-Gln-Arg-Gln-Leu-Glu-Leu-Glu-Ser-Ile-Leu-
            55                  60
Val-Ala-Leu-Leu-Gln-Arg-His-Ser-Arg-Asn-Ser-Gln-Gly.
65                  70                  75
 The resulting compound has a free amino group only at its amino terminus, which can be chemically modified. An especially preferred polypeptide compound of the invention is

desNH2-Tyr-Ala-Asp-Ala-Ile-Phe-Thr-Asn-Asn-Tyr-Arg-Arg-Val-
                        5                   10
Leu-Thr-Gln-Leu-Ser-Ala-Arg-Arg-Leu-Leu-Gln-Asp-Ile-Leu-
    15                  20                  25
Ser-Arg-Gln-Gln-Gly-Glu-Arg-Asn-Gln-Glu-Gln-Gly-Ala-Arg-
        30                  35                  40
Val-Arg-Leu-Gly-Arg-Gln-Val-Asp-Ser-Leu-Trp-Ala-Asp-Gln-
            45                  50                  55
Arg-Gln-Leu-Ala-Leu-Glu-Ser-Ile-Leu-Ala-Thr-Leu-Leu-Gln-
                60                  65
Glu-His-Arg-Asn-Ser-Gln-Gly.
70                  75

Yet another polypeptide compound of the invention is represented by the formula 
Tyr-Ala-Asp-Ala-Ile-Phe-Thr-Asn-Ser-Tyr-Arg-B-Val-Leu-
5 10
C-Gln-Leu-Ser-Ala-Arg-D-Leu-Leu-Gln-Asp-Ile-E-Asn-Arg-X;
15 20 25
 wherein B is Arg or Lys; C is Thr; D is Arg or Lys; E is Met, Val, Leu, or Ser; X is NH₂ or a chain of amino acid residues of any length. Preferred choices for X are NH₂; Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly, and Gln-Gln-Gly-Glu-Arg-Asn-Gln-Glu-Gln-Gly-Ala-Arg-Val-Arg-Leu-Gly-Arg-Gln-Val-Asp-Ser-Leu-Trp-Ala-Glu-Gln-Arg-Gln-Leu-Glu-Leu-Glu-Ser-Ile-Leu-Val-Ala-Leu-Leu-Gln-Lys-His-Ser-Arg-Asn-Ser-Gln-Gly. The latter choice is especially preferred.

Isolated DNA compounds encoding the polypeptides of the invention are also provided herein. One skilled in the art knows what codons are used to produce each amino acid. A preferred DNA compound of the invention encodes an especially preferred polypeptide compound, where A is Ala-Tyr, B is Lys, C is Thr, D is Lys, E is Leu, and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly, and is: 
ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG ATC 198
CTG GTT GCT CTG CTG CAA AAA CAT TCT CGT AAC TCC CAG GGT TAA 243
TAG 246
 wherein A is a deoxyadenyl residue, G is a deoxyguanyl residue, C is a deoxycytidyl residue and T is a thymidyl residue.

Other DNA compounds of the invention include, but are not limited to, 
ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 108
CGT AAT CAG GAA CAA GGT GCC AAG GTA CGC CTG GGT CGT CAG GTA 153
GAT TCT ATG TGG GCA GAA CAA AAG CAA ATG GAA CTG GAA CTG ATC 198
CTG GTT GCT CTG CTG CAA AAA CAT TCT CGT AAC TCC CAG GGT TAA 243
TAG 246;
 
ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG GAA 198
TCG ATC GTT GCT CTG CTG CAA AAA CAT TCT CGT AAC TCC CAG GGT 243
TAA TAG 249;
 and

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 105
CGT AAT CAG GAA CAA GGT GCC AAG GTA CGC CTG GGT CGT CAG GTA 150
GAT TCT ATG TGG GCA GAA CAA AAG CAA ATG GAA CTG GAA TCG ATC 195
CTG GTT GCT CTG CTG CAA AAA CAT TCT CGT AAC TCC CAG GGT TAA 240
TAG. 243

The following section provides a more detailed description of the present invention. For purposes of clarity and as an aid in the understanding of the invention, as disclosed and claimed herein, the following terms and abbreviations are defined below.

### Definitions

bGRF - a polypeptide with bovine growth hormone releasing factor activity.
bGRF(1-78)OH - a polypeptide comprising the carboxy-terminal 33 amino acids of the human GRF precursor.
bGRF Dimer - a polypeptide compound having two bGRF compounds linked head to tail.
bp - a base pair of double-stranded DNA.
cI857 - a gene encoding a temperature sensitive repressor of the λpL promoter.
Cloning - the process of incorporating a segment of DNA into a recombinant DNA cloning vector.
Coding Sequence - the sequence of DNA in a gene that encodes either the amino acid residue sequence of the protein expressed by the gene or, in the case of rRNA or tRNA genes, the RNA sequence of the rRNA or tRNA expressed by the gene.
Cosmid - a recombinant DNA cloning vector that can replicate in a host cell in the same manner as a plasmid but that can also utilize phage packaging mechanisms.
DHP - 2,3-dihydroxypropyl.
Gene - a segment of DNA that comprises a promoter, translational activating sequence, coding sequence, and 3' regulatory sequences positioned to drive expression of the gene product, either a protein (and thus necessarily an mRNA), tRNA or rRNA.
GRF - a polypeptide with growth hormone releasing factor activity.
GRF(1-29)NH₂ - the minimal portion of GRF that is required for full potency.
High-Level Expression in E. coli - the production of a GRF analog encoded by a cloned gene at levels such that the gene product is detectable by Coomassie Blue staining.
Isolated DNA Compound - any DNA sequence, however constructed or synthesized, which is locationally distinct from the genomic DNA of the organism in which it occurs.
pL - the leftward promoter from bacteriophage lambda.
Promoter - a DNA sequence that directs or initiates the transcription of DNA.
Recombinant DNA Cloning Vector - any autonomously replicating or integrating agent, including, but not limited to, plasmids, comprising a DNA molecule to which one or more DNA molecules can be or have been added.
Recombinant DNA Expression Vector - any autonomously replicating or integrating agent, including, but not limited to, plasmids, comprising a promoter and other regulatory sequences positioned to drive expression of a DNA segment that encodes a polypeptide or RNA.
Recombinant DNA Sequence - any DNA sequence excluding the host chromosome from which the DNA sequence is derived, which comprises a DNA sequence which has been isolated, synthesized, or partially synthesized.
Recombinant DNA Vector - any recombinant DNA cloning or expression vector.
Restriction Fragment - any linear DNA molecule generated by the action of one or more enzymes.
TcR - the tetracycline resistance-conferring gene; also used to designate the tetracycline-resistant phenotype.
Transcription Terminator - a DNA sequence that signals the termination of DNA by RNA polymerase.
Transformant - a recipient host cell that has undergone transformation.
Transformation - the introduction of DNA into a recipient host cell that changes the genotype and results in a change in the recipient cell.
Translational Activating Sequence - a regulatory DNA sequence that when transcribed into mRNA, promotes translation of mRNA into protein.

Standard three letter amino acid abbreviations are used throughout the document.

The restriction site and function maps presented in the accompanying drawings are approximate representations of the recombinant DNA vectors discussed herein. The restriction site information is not exhaustive; therefore, there may be more restriction sites of a given type on the vector than actually shown on the map.

Figure 1. A restriction site and function map of plasmid pHS190.

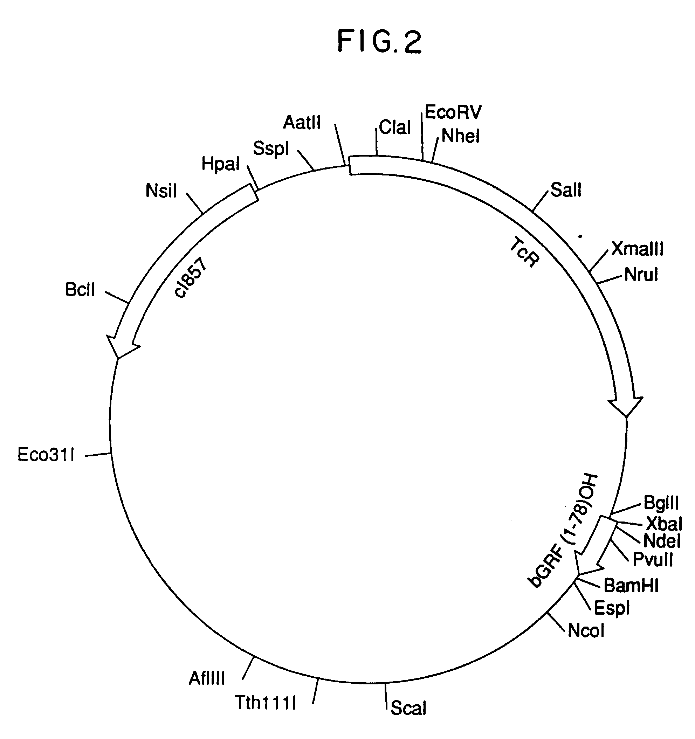

Figure 2. A restriction site and function map of plasmid pHS451.

Included in the present invention are polypeptides with growth hormone releasing factor (GRF) activity that are expressed at high levels in E. coli and that require no in vitro cleavage to be active. The compounds are also produced by any synthetic method, including enzymatic, solution, semi-synthetic and solid phase peptide synthesis. Some of the polypeptide compounds of the invention (those that begin with arginine, aspartic acid, glutamine, histidine or phenylalanine) require in vitro removal of an initiating methionine if they are to be produced by recombinant methods.

GRF is useful for stimulating the release of growth hormone. The compounds of the present invention are preferred for veterinary use, particularly in cattle, swine, sheep, poultry and fish. Also provided are DNA compounds that encode GRF polypeptides. These compounds are set out in the summary of the invention.

The polypeptides can be divided into several classes depending on the amino acids chosen for A. Compounds where A begins with methionine and X is a chain of amino acids with a length of about 31 or more are produced at high levels in E. coli. The initial methionine is cleaved by the bacterium if the second amino acid is alanine, glycine, proline or serine, yielding molecules that retain GRF activity. Compounds where A begins with Met-Pro share this characteristic. U.S. Patent 4,782,139, issued November 1, 1988, teaches compounds having the formula H-X-Pro-Peptide, wherein X is the residue of a naturally occurring amino acid, as intermediates to biologically active peptides.

Compounds where A does not begin with methionine are made by synthetic methods, including solid phase peptide synthesis or recombinant techniques if the initiator methionine is removed. The methionine is removed in vivo by E. coli if the second amino acid is alanine, glycine, proline or serine. The initiator methionine may be removed in vitro with cyanogen bromide when the methionine at position 27 is replaced by leucine, serine or valine.

The compounds with N-terminal extensions have GRF activity, although compounds where A is Met are poorly active. The activity of the N-terminally extended compounds is surprising in light of the prior art, which teaches that amino acid extensions at position 0 destroy GRF activity. Ling et al., Biochem. Biophys. Res. Commun. 122:304 (1984). The compounds may be alkylated or hydroxyalkylated to further increase the GRF activity.

Another class of polypeptide compounds has no GRF activity but is useful to create analogs that do. When A is Met the resulting compound can be expressed at high levels in E. coli. The methionine is removed to yield destyrosine compounds (compounds where the alanine of position 2 is the amino terminus) which can be used as substrates for the creation of compounds with moieties other than tyrosine at position 1. An example of a moiety that can replace tyrosine at position 1 is desNH₂-tyrosine.

All of the peptide compounds can be made by solid phase peptide synthesis or by recombinant methods. Both methods are described in U.S. Patent 4,617,149, herein incorporated by reference. Recombinant methods are preferred if a high yield is desired. A general method for the construction of any desired DNA sequence is provided in Brown et al., Methods in Enzymology 68:109 (1979), herein incorporated by reference.

Some of the polypeptide compounds of the invention can be produced at high levels in E. coli. High-level expression of the compounds of the invention can be achieved when X is a chain of amino acids of length greater than about 30. The crucial feature of the chains defined by X is their length. The chains must be of a length such that the compound escapes significant degradation by E. coli proteases. This degradation is a problem well recognized in the prior art. A sufficient chain length is estimated to be about 60 amino acids (total length of expressed compound).

Previous attempts at GRF expression in E. coli have been unsatisfactory. The first problem, encountered with direct expression, has been a very low yield of GRF. U.S. Patent 4,728,609 (Bhatt et al.). Bhatt et al. appeared to have generated approximately 25 nmoles/liter of GRF. The compounds of the present invention wherein X is an amino acid chain of length greater than about 30 have been directly expressed at levels of up to 103 µmoles/liter after in vivo removal of the initial methionine, an increase of about 4000 fold over the yield of Bhatt et al. Expression by other investigators of GRF fused with other polypeptides has yielded large amounts of the compound. However, in vitro cleavage is required to generate an active molecule, due to the fusion to another polypeptide sequence which is necessary to achieve the high expression levels. Villa et al., Eur. J. Biochem. 171:137 (1988); Geli et al., Gene, 80:129 (1989); European Patent Publication 0199018.

The present invention provides polypeptide compounds which can be directly expressed at high levels in E. coli, yet require no in vitro cleavage step to be active. The compounds of the invention where A begins with alanine, glycine, proline or serine are expressed with an initiating methionine attached, as are all proteins made in E. coli. The methionine is removed by the endogenous methionine amino peptidase of the bacterium to yield the active compound.

A preferred polypeptide of the invention has a chain of amino acids at position X derived from the human GRF precursor molecule. Gubler et al., Proc. Nat'l Acad. Sci. USA 80:4311 (1983). The activity of this molecule is surprising because precursors are generally understood to be inactive, thus allowing the cell to regulate the levels of active compound through the cleavage step. See Giraud et al., 124:1711 (1989); Fisher and Scheller, J. Biol. Chem. 263:16515 (1988); Smith and Funder, Endocr. Rev. 9:159 (1988). References concerning the GRF precursor have not suggested that it is active. Hammer et al., Nature 315:413 (1985); Mayo et al., Nature 306:86 (1983); Mayo et al., Proc. Nat'l Acad. Sci. USA 80:4311 (1983).

An especially preferred polypeptide compound of the invention comprises the carboxy-terminal portion of the human GRF precursor with leucines substituted for the methionines. This compound is 
Ala-Tyr-Ala-Asp-Ala-Ile-Phe-Thr-Asn-Ser-Tyr-Arg-Lys-Val-
0                   5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Gly-Arg-Gln-Val-Asp-Ser-Leu-Trp-Ala-Glu-Gln-
            45                  50                  55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly.
70                  75
 Alkylation of this molecule so that the free amino groups of the alanine at position 0 and the lysines at positions 12, 21, 41, 56 and 70 are substituted with 2,3-dihydroxypropyl yields a compound that is even more active and especially preferred.

Other preferred compounds of the invention are 
Ala-Tyr-Ala-Asp-Ala-Ile-Phe-Thr-Asn-Ser-Tyr-Arg-Arg-Val-
0                    5                   10

Leu-Thr-Gln-Leu-Ser-Ala-Arg-Arg-Leu-Leu-Gln-Asp-Ile-Leu-
    15                  20                  25

Asn-Arg-Gln-Gln-Gly-Glu-Arg-Asn-Gln-Glu-Gln-Gly-Ala-Arg-
        30                  35                  40

Val-Arg-Leu-Gly-Arg-Gln-Val-Asp-Ser-Leu-Trp-Ala-Glu-Gln-
            45                  50                  55

Arg-Gln-Leu-Glu-Leu-Glu-Ser-Ile-Leu-Val-Ala-Leu-Leu-Gln-
                60                  65

Arg-His-Ser-Arg-Asn-Ser-Gln-Gly;
70                  75

Pro-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Gly-Arg-Gln-Val-Asp-Ser-Leu-Trp-Ala-Glu-Gln-
            45                  50                  55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly;
70                  75
 
Pro-Tyr-Ala-Asp-Ala-Ile-Phe-Thr-Asn-Ser-Tyr-Arg-Lys-Val-
0                   5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Tyr-Ala-Asp-Ala-Ile-Phe-Thr-Asn-Ser-Tyr-Arg-
            45                  50                  55

Lys-Val-Leu-Thr-Gln-Leu-Ser-Ala-Arg-Lys-Leu-Leu-Gln-Asp-
                60                  65

Ile-Leu-Asn-Arg-Gln-Gln-Gly-Glu-Arg-Asn-Gln-Glu-Gln-Gly-
70                  75                  80

Ala-Lys-Val-Arg-Leu;
    85

Met-Ala-Tyr-Ala-Asp-Ala-Ile-Phe-Thr-Asn-Ser-Tyr-Arg-Lys-
    0                   5                   10

Val-Leu-Thr-Gln-Leu-Ser-Ala-Arg-Lys-Leu-Leu-Gln-Asp-Ile-
        15                  20                  25

Leu-Asn-Arg-Gln-Gln-Gly-Glu-Arg-Asn-Gln-Glu-Gln-Gly-Ala-
            30                  35                  40

Lys-Val-Arg-Leu-Gly-Arg-Gln-Val-Asp-Ser-Leu-Trp-Ala-Glu-
                45                  50

Gln-Lys-Gln-Leu-Glu-Leu-Glu-Ser-Ile-Leu-Val-Ala-Leu-Leu-
55                  60                  65

Gln-Lys-His-Ser-Arg-Asn-Ser-Gln-Gly;
    70                  75
 
Met-Ala-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-Ile-
15 20 25

Met-Asn-Arg-Gln-Gln-Gly-Glu-Arg-Asn-Gln-Glu-Gln-Gly-Ala-
30 35 40

Lys-Val-Arg-Leu-Gly-Arg-Gln-Val-Asp-Ser-Met-Trp-Ala-Glu-
45 50

Gln-Lys-Gln-Met-Glu-Leu-Glu-Ser-Ile-Leu-Val-Ala-Leu-Leu-
55 60 65

Gln-Lys-His-Ser-Arg-Asn-Ser-Gln-Gly;
70 75

Pro-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-
15 20 25

Ile-Met-Asn-Arg-Gln-Gln-Gly-Glu-Arg-Asn-Gln-Glu-Gln-
30 35

Gly-Ala-Lys-Val-Arg-Leu-Gly-Arg-Gln-Val-Asp-Ser-Met-
40 45 50

Trp-Ala-Glu-Gln-Lys-Gln-Met-Glu-Leu-Glu-Ser-Ile-Leu-
55 60

Val-Ala-Leu-Leu-Gln-Lys-His-Ser-Arg-Asn-Ser-Gln-Gly; and
65 70 75

desNH2-Tyr-Ala-Asp-Ala-Ile-Phe-Thr-Asn-Asn-Tyr-Arg-Arg-Val-
5 10
Leu-Thr-Gln-Leu-Ser-Ala-Arg-Arg-Leu-Leu-Gln-Asp-Ile-Leu-
15 20 25
Ser-Arg-Gln-Gln-Gly-Glu-Arg-Asn-Gln-Glu-Gln-Gly-Ala-Arg-
30 35 40
Val-Arg-Leu-Gly-Arg-Gln-Val-Asp-Ser-Leu-Trp-Ala-Asp-Gln-
45 50 55
Arg-Gln-Leu-Ala-Leu-Glu-Ser-Ile-Leu-Ala-Thr-Leu-Leu-Gln-
60 65
Glu-His-Arg-Asn-Ser-Gln-Gly.
70 75
 Alkylated and hydroxyalkylated versions of these compounds are also preferred, especially where the free amino groups are replaced with 2,3-dihydroxypropyl.

Included in the compounds of this invention are their pharmaceutically acceptable non-toxic acid addition salts and their pharmaceutically acceptable non-toxic carboxylic acid salts.

The term "pharmaceutically acceptable non-toxic acid addition salts" encompasses both organic and inorganic acid addition salts including, for example, those prepared from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. Preferably, the acid addition salts are those prepared from hydrochloric acid, acetic acid, or succinic acid. Any of the above salts is prepared by conventional methods.

The term "carboxylic acid salts" includes, for example, amine, ammonium, quaternary ammonium, alkali metal and alkaline earth metal salts such as calcium, magnesium, sodium, potassium, and lithium, and the like.

The present invention also provides a pharmaceutical composition comprising as the active agent a polypeptide compound or a pharmaceutically acceptable acid or carboxylic acid addition salt thereof, wherein said polypeptide compound has the formula 
A-Ala-Asp-Ala-Ile-Phe-Thr-Asn-R-Tyr-
5 10
Arg-B-Val-Leu-C-Gln-Leu-Ser-Ala-Arg-D-Leu-Leu-
15 20
Gln-Asp-Ile-E-Asn-Arg-X;
25
 wherein A is Ala-Tyr, desNH₂-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, and Met-Ser-Pro-Tyr; R is Asn or Ser; B is Arg or Lys; C is Gly, Ala, Leu, Val, Ile, or Thr; D is Arg or Lys, E is Met, Val, Leu, or Ser; X is NH₂ or one or more amino acids; and none, one or more than one of the amino acids having a free amino group is chemically modified and a pharmaceutically acceptable solid or liquid carrier.

Also provided in the present invention is a method for inducing growth hormone release which comprises administering an effective amount of a polypeptide compound represented by the formula 
A-Ala-Asp-Ala-Ile-Phe-Thr-Asn-R-Tyr-
5 10
Arg-B-Val-Leu-C-Gln-Leu-Ser-Ala-Arg-D-Leu-Leu-
15 20
Gln-Asp-Ile-E-Asn-Arg-X;
25
 wherein A is Ala-Tyr, desNH₂-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, and Met-Ser-Pro-Tyr; R is Asn or Ser; B is Arg or Lys; C is Gly, Ala, Leu, Val, Ile, or Thr; D is Arg or Lys, E is Met, Val, Leu, or Ser; X is NH₂ or one or more amino acids; and none, one or more than one of the amino acids having a free amino group is chemically modified.

The polypeptide compounds of the invention may be assayed in numerous systems. One in vivo assay is carried out in sodium pentobarbital-anesthetized rats. Wehrenberg et al., Biochem. Biophys. Res. Commun. 109:382 (1982). Growth hormone release is measured in vitro with rat anterior pituitary cells. Heiman et al., Endocrinol. 116:410 (1985). Assays are also performed in wether lambs as described in the Examples provided hereinafter.

The DNA compounds of the present invention encode polypeptide compounds of the invention. It is well known in the art how to construct a DNA sequence that will encode for a given amino acid sequence. To produce the desired polypeptide, one must merely insert the DNA sequence in any one of many recombinant DNA expression vectors. The coding sequence must be operably linked to a promoter and ribosome binding site that function in the host cell. A preferred vector is derived from E. coli plasmid pHS190, which comprises the TcR gene, the lambda cI857 repressor and the lambda pL promoter. Plasmid pHS190 is on deposit with the Northern Regional Research Laboratories under the accession number NRRL B-18410 (date of deposit: September 9, 1988).

The desired DNA sequence is constructed so that it has an XbaI site at the 5' end of the coding strand and a BamHI site at the 3' end of the coding strand. To generate the vector DNA, pHS190 is digested with EcoRI. The protruding ends are then filled in and the plasmid is religated. Subsequent digestion with BamHI and XbaI yields the vector plasmid. The constructed GRF coding sequence can then be inserted into the vector. The resulting plasmid is designated pHS451 when the inserted DNA sequence is the preferred DNA sequence of the invention. This sequence is

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG ATC 198
CTG GTT GCT CTG CTG CAA AAA CAT TCT CGT AAC TCC CAG GGT TAA 243
TAG. 246

Other DNA sequences encoding polypeptide compounds of the invention are: 
ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 108
CGT AAT CAG GAA CAA GGT GCC AAG GTA CGC CTG GGT CGT CAG GTA 153
GAT TCT ATG TGG GCA GAA CAA AAG CAA ATG GAA CTG GAA CTG ATC 198
CTG GTT GCT CTG CTG CAA AAA CAT TCT CGT AAC TCC CAG GGT TAA 243
TAG 246
ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG GAA 198
TCG ATC GTT GCT CTG CTG CAA AAA CAT TCT CGT AAC TCC CAG GGT 243
TAA TAG 249
 and

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 105
CGT AAT CAG GAA CAA GGT GCC AAG GTA CGC CTG GGT CGT CAG GTA 150
GAT TCT ATG TGG GCA GAA CAA AAG CAA ATG GAA CTG GAA TCG ATC 195
CTG GTT GCT CTG CTG CAA AAA CAT TCT CGT AAC TCC CAG GGT TAA 240
TAG. 243

Transformation of the ligated plasmid into an E. coli strain results in a recombinant cell capable of expressing a polypeptide compound of the invention. The cell is grown at 32°C until it is desired to express the polypeptide compound. The cI857 repressor becomes inactivated upon a shift in growth temperature to 42°C and the pL promoter is derepressed. A large amount of polypeptide having GRF activity is then produced at levels up to about 15% of total cell protein. The active polypeptide can be purified by techniques well-known to skilled artisans and set out in the following Examples.

In administering the polypeptide compounds of this invention parenterally, the pharmaceutical forms suitable for injection include sterile aqueous solutions or dispersions and sterile powders for reconstitution into sterile injectable solutions or dispersions. The carrier can be a solvent or dispersing medium containing, for example, water, ethanol, polyol (for example glycerol, propylene glycol, liquid polyethylene glycol, and the like), suitable mixtures thereof, and vegetable oils. Proper fluidity can be maintained, for example, by the use of a coating such as lecithin, by the maintenance of the required particle size in the case of dispersion and by the use of surfactants. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin.

Sterile injectable solutions can be prepared by incorporating the compounds of this invention in the required amount of the appropriate solvent with various of the other ingredients, as desired. If desired, and for more effective distribution, the compounds can be incorporated into slow release or targeted delivery systems such as polymer matrices, liposomes, and microspheres. The compounds may be delivered via mechanical release devices, osmotic pumps, or any other release device or system which provides continuous or pulsatile delivery.

Doses of the compounds of this invention are administered to the recipient for a period during which stimulation of the release of growth hormone is desired. The weight of the recipient and mode of administration will have an influence upon the size of the dose necessary to induce a particular response. Preferred doses in sheep are about 0.1-3.0 mg/day; a most preferred dose is about 1.0 mg/day. For cattle preferred doses are about 0.5-12 mg/day. An especially preferred dose is 3 mg/day.

It is especially advantageous to formulate the compounds of this invention in unit dosage form for ease of administration and uniformity of dosage. Unit dosage form as used herein refers to physically discrete units suited as unitary dosages for the subject to be treated. Each unit contains a predetermined quantity of the compound calculated to produce the desired therapeutic effect in association with the pharmaceutically acceptable carrier. The specific unit dosage form is dictated by and directly dependent upon (a) the unique characteristics of the particular composition and (b) the particular therapeutic effect to be achieved.

The following examples are illustrative of this invention.

### Example 1

### Construction of Plasmid pHS451

Plasmid pHS451 is a recombinant DNA expression vector which produces large amounts of the preferred polypeptide compound of the invention when the host cell is cultured under the proper conditions.

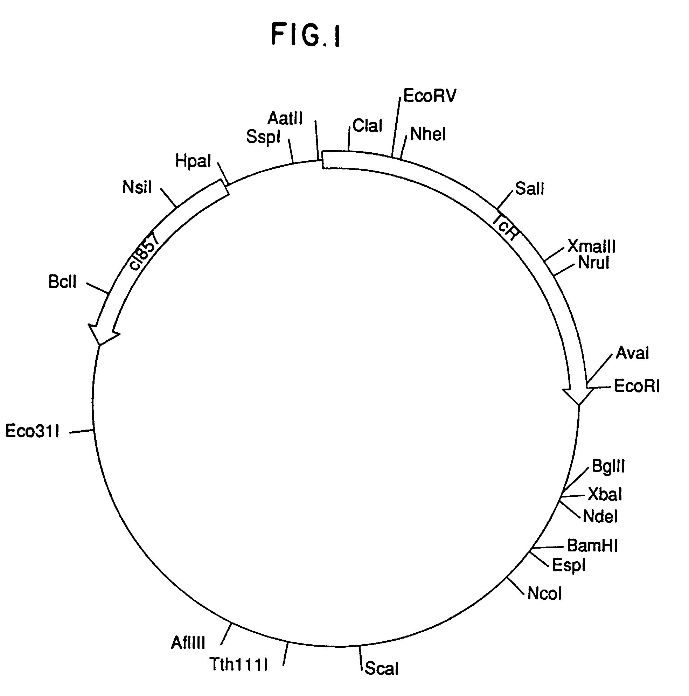

A lyophil of E. coli K12 RV308/pHS190 can be obtained from the Northern Regional Research Laboratories (NRRL), Peoria, IL 61604, under the accession number NRRL B-18410 (date of deposit: September 9, 1988) and used directly as the "culture" in the process below. A restriction site and function map of plasmid pHS190 is presented in Figure 1 of the accompanying drawings. Ten ml of TY broth (10 g tryptone, 5 g NaCl, and 5 g yeast extract per liter) containing 5 pg/ml of tetracycline is inoculated with a culture of E. coli K12 RV308/pHS190 and incubated with aeration at 30°C overnight (15-18 hours). The resulting culture is used as a source of plasmid pHS190.

One liter of TY broth containing 5 pg/ml tetracycline is inoculated with a culture of E. coli K12 RV308/pHS190 and incubated with aeration at 32°C overnight (15-18 hours). The culture is then centrifuged at 5200 rpm in a Sorvall (DuPont Co., Instrument Products, Biomedical Division, Newtown, CT 06470) GSA rotor for 10 minutes at 4°C to pellet the cells. The resulting supernatant is discarded. The cell pellet is resuspended in 28 ml of a solution of 25% sucrose and 50 mM EDTA (ethylenediamine tetraacetic acid). About 1 ml of a solution of 20 mg/ml lysozyme in 0.25 M Tris-HCl (tris(hydroxymethyl)aminomethane hydrochloride), pH = 8.0, and about 1.5 ml of 0.5 M EDTA, pH = 8.0, are added to and mixed with the cell suspension. The resulting mixture is incubated on ice for 15 minutes. Three ml of lysing solution (prepared by mixing 3 ml of 10% Triton® X-100 (Rohm & Haas); 75 ml of 0.25 M EDTA, pH = 8.0; and 7 ml of water) are added to the lysozyme-treated cells with gentle mixing. The resulting solution is incubated on ice for another 15 minutes.

The cellular debris is removed from the solution by centrifugation at 17,000 rpm in a Sorvall SS34 rotor for about 45 minutes at 4°C. About 28.6 g of CsCl and ∼1 ml of a 5 mg/ml ethidium bromide solution are added to the ∼30 ml of supernatant. Then, the volume is adjusted to 40 ml with water and the solution decanted into an ultracentrifuge tube. The tube is sealed, and the solution is centrifuged at 49,500 rpm in a Ti70 rotor (Beckman, 7360 N. Lincoln Avenue, Lincolnwood, IL 60646) for ∼18 hours. The resulting plasmid band, visualized with ultraviolet light, is isolated, extracted with CsCl-saturated isopropanol to remove the ethidium bromide, and dialysed against three changes of ∼20 volumes of TE buffer (10 mM Tris-HCl, pH = 7.5, and 1 mM EDTA). The dialysate is collected; then, two volumes of ethanol and 0.05 volumes of 3 M sodium acetate solution are added. The ethanol mixture is cooled to -20°C, and the plasmid DNA is pelleted by centrifugation at 10,000 rpm for 30 minutes in an SS34 rotor at -10°C. The resulting pellet is resuspended in ∼1 ml of TE buffer and then extracted with an equal volume of a phenol:chloroform mixture (1:1, v/v). The DNA in the aqueous phase is recovered by the addition of 0.1 volume of 3 M sodium acetate and 2 volumes of ethanol, followed by incubation at -20°C for ∼30 minutes and centrifugation at 15,000 rpm in an SS34 rotor for 20 minutes. The resulting DNA pellet is rinsed first with 70% ethanol and then with 100% ethanol and dried.

The ∼1.0 mg of plasmid pHS190 DNA obtained by the above procedure is suspended in 1.5 ml of 0.1X TE buffer and stored at -20°C. About 10 pg of plasmid pHS190 DNA are digested with restriction enzyme EcoRI (∼10 units) in a reaction containing the DNA in EcoRI buffer (100 mM Tris-HCl, pH = 7.5, 5 mM MgCl₂, 50 mM NaCl). The reaction is incubated for 2 hours at 37°C.

To convert the 5' overhanging ends to blunt ends 0.5 mM of each dNTP (dATP, dCTP, dGTP, and TTP) is added along with 1-5 units Klenow fragment (Boehringer Mannheim Biochemicals, 7941 Castleway Dr., P.O. Box 50816, Indianapolis, IN 46250). The reaction is incubated at 30°C for 15 minutes, then the enzyme is inactivated by treatment at 75°C for 10 minutes. The reaction mixture is extracted with phenol, phenol/chloroform, chloroform and then ethanol precipitated.

The plasmid is then resuspended in 50 µl of a solution containing 40 mM Tris HCl, pH 7.5, 10 mM MgCl₂, 10 mM dithiothreitol (DTT), 0.5 mM adenosine triphosphate, and 1 U T4 DNA ligase (Boehringer-Mannheim Biochemicals, 7941 Castleway Drive, Indianapolis, IN 46250). The reaction is incubated at 14°C overnight.

The ligated mixture was transformed into E. coli K12 RV308 (available from the NRRL as NRRL B-15624; date of deposit: September 28, 1983) by the following procedure from Maniatis et al., Molecular Cloning 250-251 (1982). One hundred ml of TY broth in a 500-ml flask was inoculated with one ml of an overnight culture of RV308. The cells were grown with vigorous shaking at 37°C to a density of ∼5 x 10⁷ cells/ml. The culture was placed on ice for ten minutes, then centrifuged at 4000 xg for 5 minutes at 4°C. The cells were resuspended in 50 ml ice-cold 50 mM CaCl₂ in 10 mM Tris-HCl, pH = 8.0. The cells were again incubated on ice for 15 minutes and recentrifuged. The cells were then resuspended in 3.3 ml of the calcium chloride solution. The ligation mixture was added to 200 pl of cells and incubated on ice for 30 minutes. The cells were then transferred to a 42°C water bath for 2 minutes. One ml of TY broth was added to the tube and the cells were incubated for one hour at 30°C. Two hundred µl aliquots were then plated onto TY agar (TY broth + 1.5% agar) plates containing 5 µg/ml tetracycline and grown overnight at 30°C.

The plasmid is then resuspended in 10 µl of water. The vector is generated by digesting the plasmid with XbaI and BamHI. About 1 pl of XbaI (∼10 units) is added to 10 pl plasmid DNA (∼10 pg) and 5 pl 10X XbaI buffer (500 mM Tris-HCl, pH = 8.0, 100 mM MgCl₂, and 500 mM NaCl). After incubation at 37°C for 90 minutes, 0.5 pl of 5 M NaCl and 1 µl BamHI (∼10 units) are added and the incubation continued at 37°C for an additional 90 minutes. The reaction mixture is then subjected to agarose gel electrophoresis, and the ∼5.75 kb XbaI-BamHI vector fragment is isolated by electroelution followed by ethanol precipitation.

The following DNA sequence was synthesized on an Applied Biosystems Model 380B synthesizer using techniques well-known to one skilled in the art. The division of the sequence into oligonucleotides was done by the procedure of Brown et al., Methods in Enzymology 68:109 (1979). The DNA sequence encodes a preferred polypeptide of the invention wherein A is Met-Ala-Tyr, B is Lys, C is Thr, D is Lys, E is Leu and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly.

CTAGAGGGTATTAATAATGTATATTGATTTTAATAAGGAGGAATAATCATATGGCTTATG
1868 --+---------+---------+---------+---------+---------+-------
         TCCCATAATTATTACATATAACTAAAATTATTCCTCCTTATTAGTATACCGAATAC

CTGATGCTATATTTACTAATTCTTATCGTAAAGTACTTACTCAGCTGTCTGCACGTAAAC
1928 --+---------+---------+---------+---------+---------+-------
     GACTACGATATAAATGATTAAGAATAGCATTTCATGAATGAGTCGACAGACGTGCATTTG

TGCTACAAGATATCCTGAACCGTCAACAGGGTGAACGTAATCAGGAACAAGGTGCCAAGG
1988 --+---------+---------+---------+---------+---------+-------
     ACGATGTTCTATAGGACTTGGCAGTTGTCCCACTTGCATTAGTCCTTGTTCCACGGTTCC

TACGCCTGGGTCGTCAGGTAGATTCTCTGTGGGCAGAACAAAAGCAACTTGAACTGGAAT
2048 --+---------+---------+---------+---------+---------+-------
     ATGCGGACCCAGCAGTCCATCTAAGAGACACCCGTCTTGTTTTCGTTGAACTTGACCTTA

CGATCCTGGTTGCTCTGCTGCAAAAACATTCTCGTAACTCCCAGGGTTAATAG
2108 --+---------+---------+---------+---------+---------+
     GCTAGGACCAACGAGACGACGTTTTTGTAAGAGCATTGAGGGTCCCAATTATCCTAG

The DNA comprising the coding sequence was then mixed with and ligated to the XbaI-BamHI vector fragment constructed above. The ligated mixture was then transformed in E. coli K12 RV308 as described above. The plasmid DNA of tetracycline-resistant transformants was analyzed by restriction enzyme digestion to verify that the plasmid was pHS451.

### Example 2

### Purification of E. coli-produced GRF Analogs

### A. Expression of the GRF Analog in E. coli

E. coli K12 RV308/pHS451 was grown in TY broth containing 5 µg/ml tetracycline at 32°C until the cells reached mid-log phase. The temperature of the mixture was raised to 42°C and incubation continued for about 3 more hours. The cI857 temperature-sensitive repressor of the lambda pL promoter, positioned to drive GRF analog expression on plasmid pHS451, is inactivated at 42°C, thus allowing the expression of the GRF analog.

### B. Isolation of Granules Containing the GRF Analog

Granules containing the GRF analog are isolated. First, the whole cells are centrifuged, then resuspended in water at 10°C. The cell slurry is homogenized in a Gaulin homogenizer at 8000 psig. The homogenized slurry is then diluted in water and agitated for 10 minutes, followed by adjustment of the pH to 8.4-8.6 with 10% sodium hydroxide. The mixture is then centrifuged. The solids represent the GRF analog-containing granules, which are frozen at -70°C until further use.

### C. Final Purification of the GRF Analog

The granules prepared in Example 2B are thawed at 2-5°C. The granules are solubilized by the addition of ten volumes of 0.05 N acetic acid-7M urea followed by homogenization for 5-8 minutes. The pH is then adjusted to 2.5-2.6 by the addition of 10% hydrochloric acid. The mixture is agitated 12-15 hours at 2-5°C. The solution is clarified by filtration through a Sparkler filter coated with Dicalite Speedex filter aid (Grefco, Torrance, CA). The conductivity of the solution is reduced to less than 4000 µohms by dilution with the acetic acid-urea solution.

A cation exchange column is prepared using S Sepharose® (Pharmacia, 800 Centennial Ave., Piscataway, NJ 08854) resin. The column contains one liter of resin per 50 g of material. The material is added to the column at a flow rate of 0.1 l/cm²/hr and washed with 2 column volumes of 0.1 M sodium chloride in the acetic acid-urea solution. The GRF analog is eluted by a linear gradient of 0.25 M to 1.6 M sodium chloride in acetic acid-urea using three column volumes of each with 0.1 column volume fractions collected. The GRF analog-containing fractions are identified by conductivity, O.D.₂₇₆, HPLC and polyacrylamide gel electrophoresis. The fractions are then pooled.

An equal volume of acetic acid-urea solution is added to the pooled fractions. The material is then applied to a column containing S Sepharose® resin in acetic acid-urea sized to accommodate 50 g of protein per liter of resin. The flow rate is 0.02 l/cm²/hr. The GRF analog fractions are eluted by a linear gradient of 0.25 M to 1.2 M sodium chloride in acetic acid-urea. Fractions of 0.1 column volume are collected. The fractions are analyzed as before and the GRF analog-containing fractions are pooled.

A Sephadex® G-15 (Pharmacia) column is prepared in 0.02 M glycine, pH 2.5 with a column volume five times the volume of the previously pooled fractions. Fractions containing the O.D.₂₇₆ peak are isolated.

A column containing SP20SS resin (Sephabeads, Mitsubishi Chemical, Tokyo) in 10% acetonitrile-0.02 M glycine, pH 2.5, is then prepared. The pooled GRF analog-containing solution is made 10% in acetonitrile and added to the column at a flow rate of 1.5-2 column volumes per hour. The column is washed with 2 column volumes of the acetonitrile-glycine buffer. The GRF analog is eluted by a gradient formed by three column volumes of 10% acetonitrile-0.02 M glycine mixed with three column volumes 50% acetonitrile-0.02 M glycine. Fractions of 0.1 column volume are collected and assayed for the GRF analog.

The GRF analog-containing material is then chromatographed over a Sephadex® G15 column equilibrated in 0.25 M acetic acid. The O.D.₂₇₆ peak is then isolated and lyophilized until further use.

### Example 3

### Dihydroxypropylation of the GRF Analog

A portion of the GRF analog prepared in Example 2 is dihydroxypropylated by treatment with glyceraldehyde in the presence of sodium cyanoborohydride in substantial accordance with the procedure of Acharya and Manjula, Biochemistry 26:3524 (1987).

Five grams of the analog prepared in Example 2 is dissolved in 60 ml of 25% 1-propanol in 0.1% trifluoroacetic acid (Pierce, Rockford, IL) at room temperature with stirring. The pH is adjusted to 8 with ∼1.7 ml 5N sodium hydroxide. To this is added 0.92 g DL-glyceraldehyde (Sigma Chemical Co., P.O. Box 14508, St. Louis, MO 63178) and 0.85 g sodium cyanoborohydride (Aldrich, Milwaukee, WI). The mixture is stirred for one hour at room temperature. A 9X65 cm Sephadex® G-10 (Pharmacia, 800 Centennial Avenue, Piscataway, NJ 08854) column is packed in water. The material containing the GRF compound is then washed into the column with 10 ml 25% propanol. The column is eluted with about 1500 ml H₂O. Fractions of 24 ml are collected while monitoring the UV fluorescence at 280 nanometers. After the fiftieth fraction is collected the eluting buffer is changed from H₂O to 10% acetic acid. The peak fractions are pooled and lyophilized.

### Example 4

### Assay of the GRF Analog and its Alkylated Form in Wether Lambs

Twelve wether lambs weighing approximately 65 kg each were fitted with a sub-cutaneous cannula behind the right shoulder and a jugular vein cannula for blood withdrawal on the left side. The infusate was administered in the sub-cutaneous cannula by an Infu-Check™ (IVAC Corporation, P.O. Box 85335, San Diego, CA 92121-1579) pump at a rate of 2.0 ml/hr. The duration of infusion was 120 hours. The peptides were dissolved in 5 ml of 0.01 M H₃PO₄ and diluted to 1805 ml with NaH₂PO₄ buffer containing 0.1% ovine serum albumin and 50 pg/ml polymyxin B. The infusate contained 20.8 pg/ml of peptide. The dosage was 1.0 mg analog/lamb/day. There were two treatment groups. One group was treated with the most preferred compound of the invention where A is Ala, B is Lys, C is Thr, D is Lys, E is Leu and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly. This compound is designated Ala⁰ in Table 1. The second treatment group was treated with the same compound except that the free amino groups were monoalkylated to 2,3-dihydroxypropyl. This compound is labelled DHP Ala⁰ in Table 1.

The lambs were bled four times daily via the jugular cannula starting 24 hours prior to the initiation of treatment and continuing throughout treatment and for 24 hours after treatment was terminated. The blood samples were collected one half hour before each feeding and one half hour after each feeding. Plasma samples were assayed for growth hormone release.

The lambs were fed 400 g of a high energy ration at 0700 and 1500 hours each day. The results, as shown in the tables below, indicate that the compounds of the invention have growth hormone releasing activity. Preferred compounds of the invention, prepared in the previous Example, gave the results disclosed in Table 1. Other compounds of the invention are listed in subsequent tables.

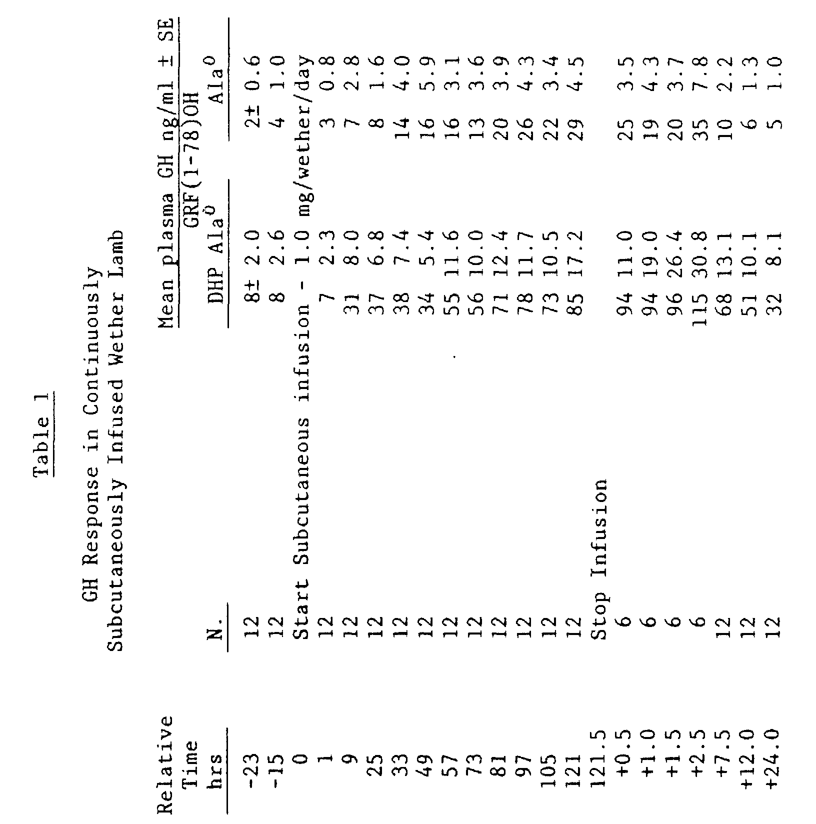

The following tables provide the results of growth hormone release assays in the wether lamb with various GRF analogs of the present invention. The analogs were generated by the solid phase synthesis method as described in U.S. Patent 4,617,149, herein incorporated by reference. The analogs are bovine GRF with different amino acid substitutions and extensions as listed.

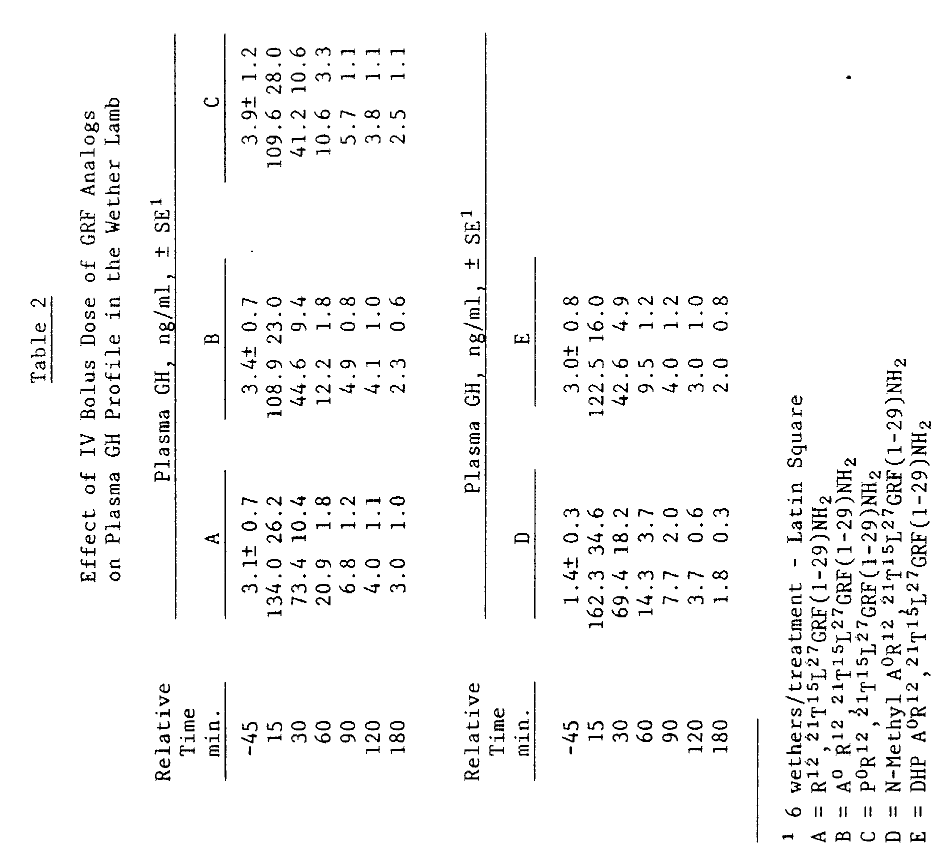

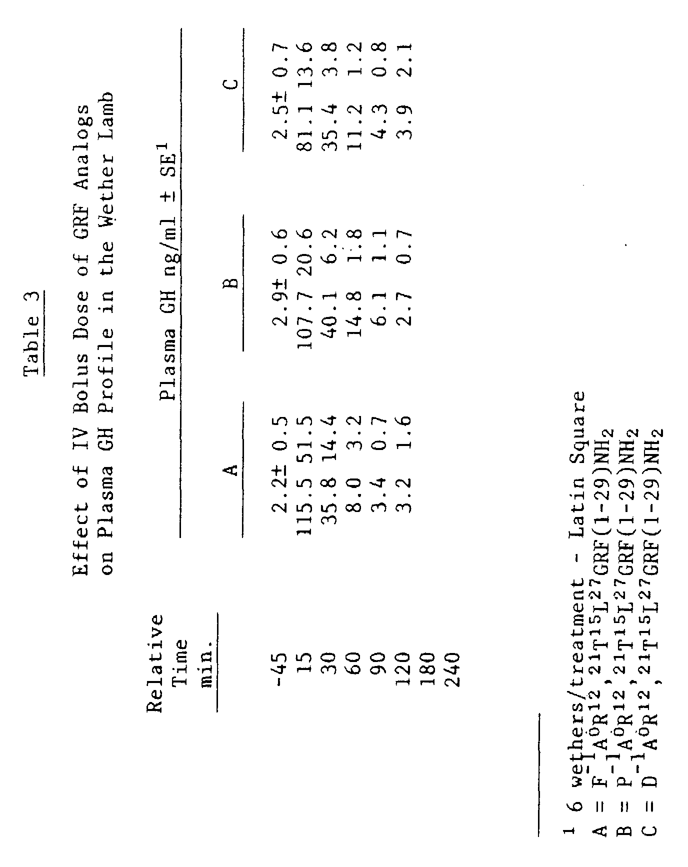

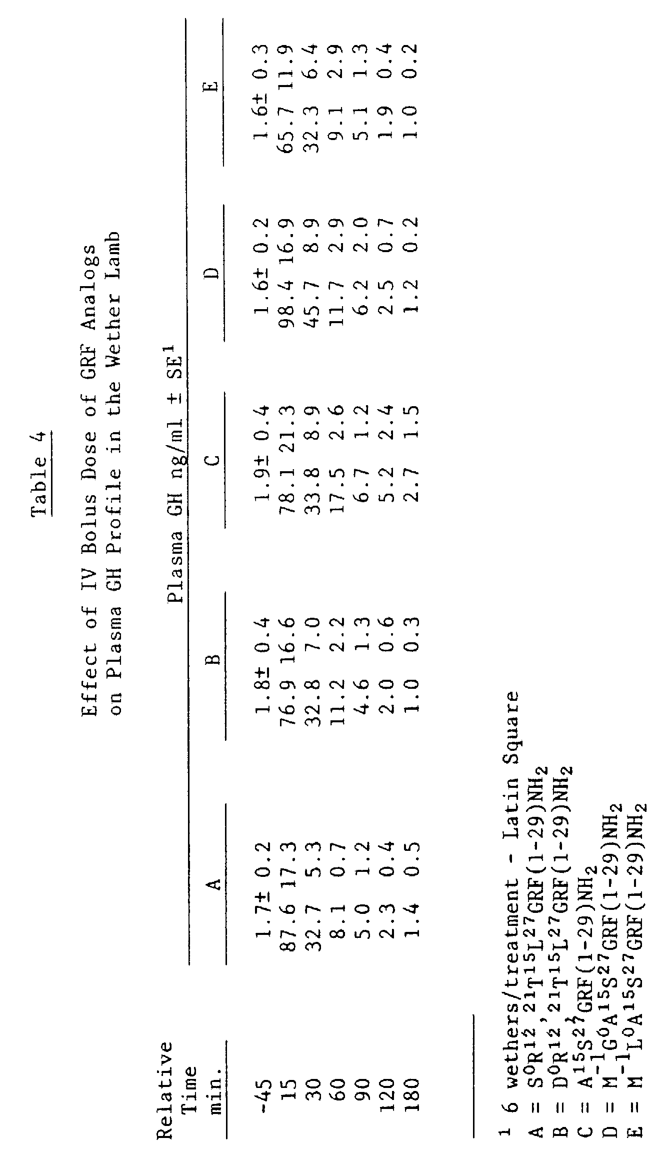

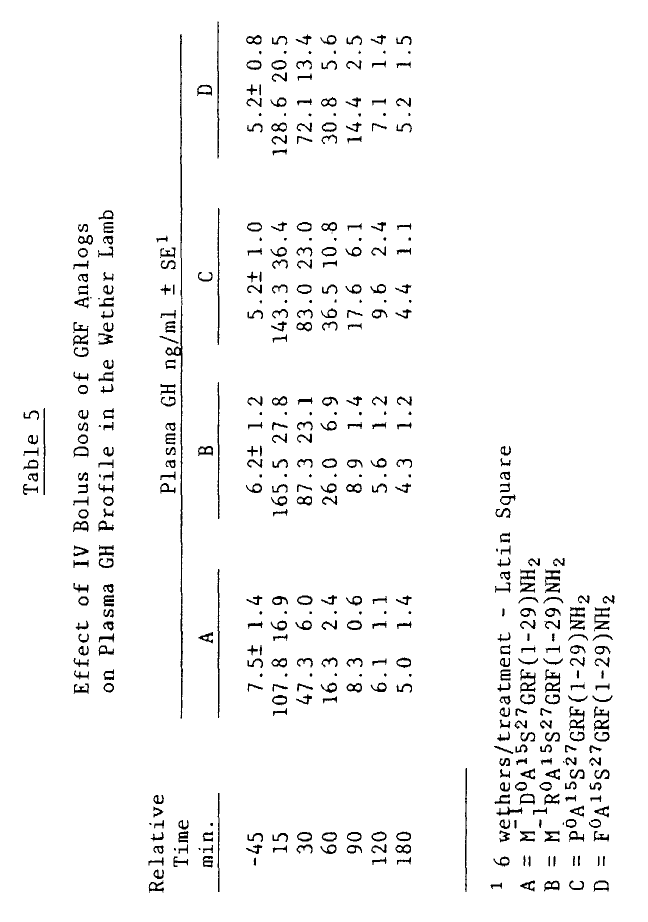

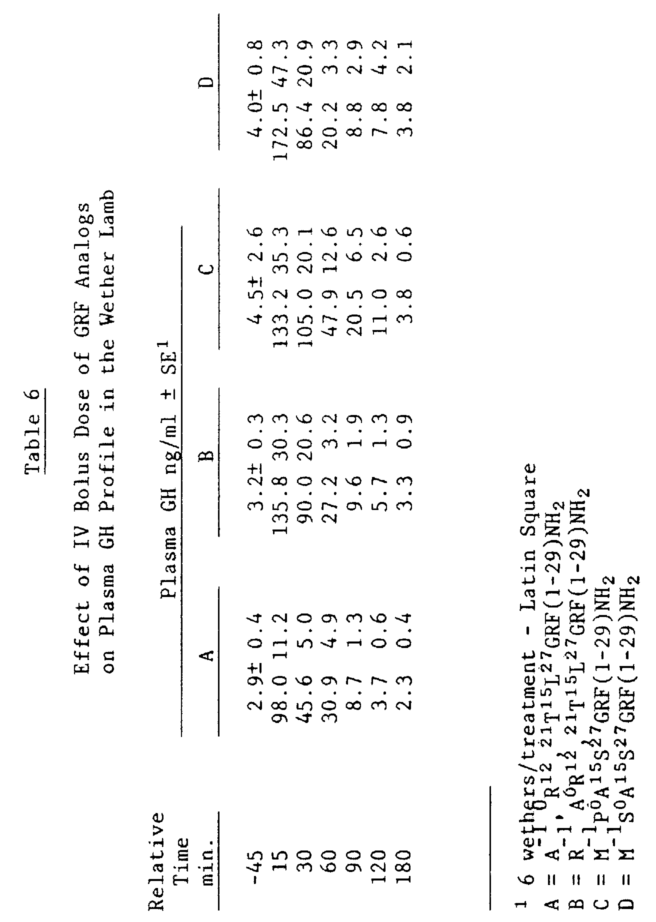

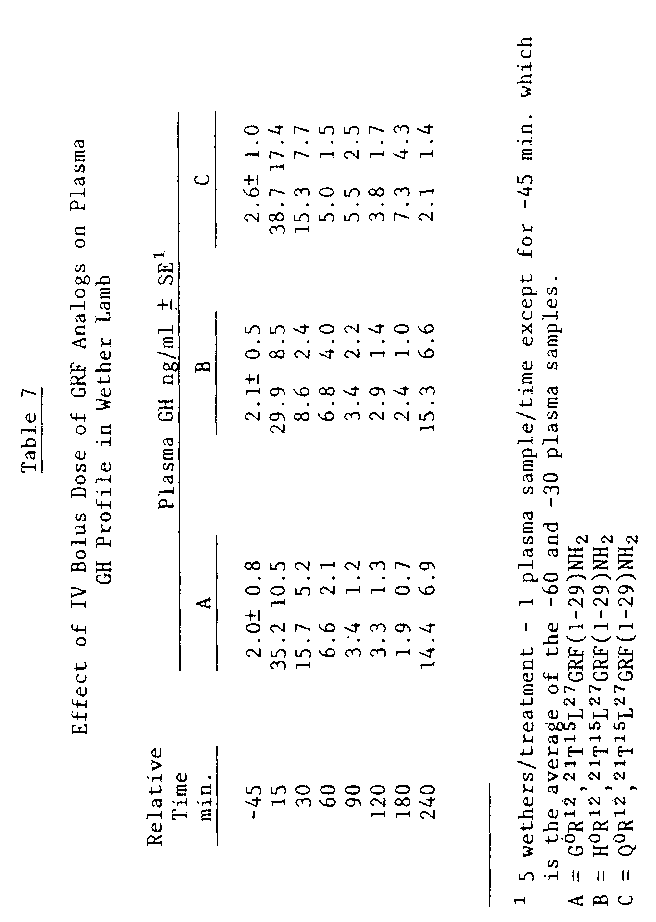

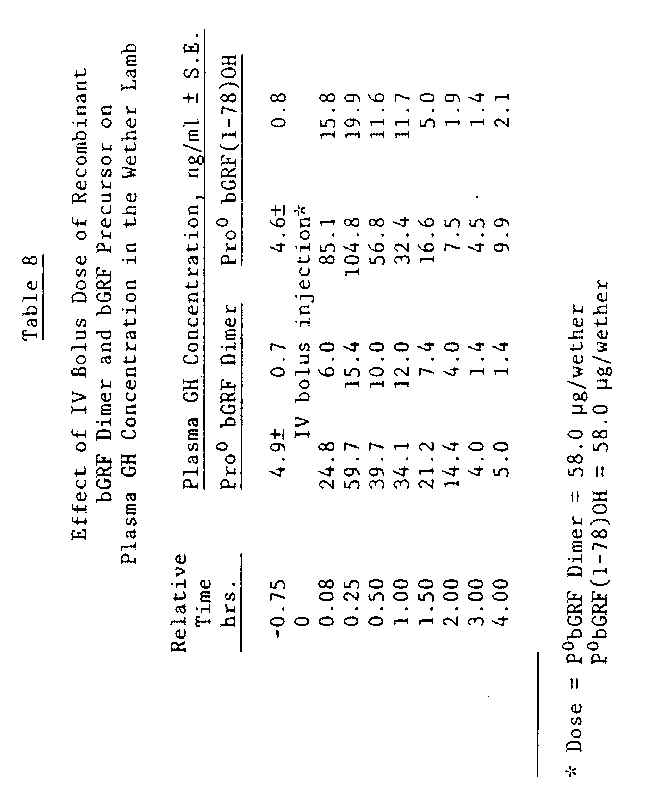

### Example 5

Various bGRF peptide analogs were synthesized by the solid phase synthesis method as described in U.S. Patent 4,617,149. The compounds were assayed in rat pituitary cells by the method of Heiman et al., Endocrinol. 116:410 (1985). The analogs are based on the sequence of the first 29 amino acids of human growth hormone releasing factor with the substitutions listed.

**Table 9**

| Potency of GRF-analog Mediated GH Secretion from Rat Pituitary Cells^{a} | | |
|---|---|---|
| Analog | Potency^{b} nM (mean;n) | Relative Potency^{c} (% of std^{d}) |
| hGRF (1-44)0H | .136; 3 | 1.0 |
| [R¹²,T¹⁵,R²¹,L²⁷]hGRF(1-29)NH₂ | .136; 10 | 1.0 |
| DHP[R¹²,T¹⁵,R²¹,L²⁷]hGRF(1-29)NH₂ | .17; 1 | .8 |
| [P⁰,R¹²,T¹⁵,R²¹,L²⁷]hGRF(1-29)NH₂ | .665; 2 | .20 |
| DHP[P⁰,R¹²,T¹⁵,R²¹,L²⁷]hGRF(1-29)NH₂ | 1.01; 1 | .13 |
| [A⁰,R¹²,T¹⁵,R²¹,L²⁷]hGRF(1-29)NH₂ | .534; 9 | .255 |
| DHP[A⁰,R¹²,T¹⁵,R²¹,L²⁷]hGRF(1-29)NH₂ | .406; 2 | .335 |
| [N-Me-A⁰,R¹²,T¹⁵,R²¹,L²⁷]hGRF(1-29)NH₂ | .78; 3 | .17 |
| [N-Ac-A⁰,R¹²,T¹⁵,R²¹,L²⁷]hGRF(1-29)NH₂ | 1.40; 2 | .10 |
| [Q⁰,R¹²,T¹⁵,R²¹,L²⁷]hGRF(1-29)NH₂ | .712; 2 | .191 |
| [G⁰,R¹²,T¹⁵,R²¹,L²⁷]hGRF(1-29)NH₂ | .337; 2 | .36 |
| [H⁰,R¹²,T¹⁵,R²¹,L²⁷]hGRF(1-29)NH₂ | .528; 1 | .26 |
| [D⁰,R¹²,T¹⁵,R²¹,L²⁷]hGRF(1-29)NH₂ | .56; 2 | .24 |
| [S⁰,R¹²,T¹⁵,R²¹,L²⁷]hGRF(1-29)NH₂ | .51; 2 | .27 |
| [P^{⁻1},P⁰,R¹²,T¹⁵,R²¹,L²⁷]hGRF(1-29)NH₂ | 1.45; 1 | .09 |
| DHP[P^{⁻1},P⁰,R¹²,T¹⁵,R²¹,L²⁷]hGRF(1-29)NH₂ | .959; 1 | .14 |
| [P^{⁻1},A⁰,R¹²,T¹⁵,R²¹,L²⁷]hGRF(1-29)NH₂ | 1.01; 2 | .13 |
| [R^{⁻1},A⁰,R¹²,T¹⁵,R²¹,L²⁷]hGRF(1-29)NH₂ | .885; 1 | .15 |
| [A^{⁻1},A⁰,R¹²,T¹⁵,R²¹,L²⁷]hGRF(1-29)NH₂ | .526; 2 | .26 |
| [F^{⁻1},A⁰,R¹²,T¹⁵,R²¹,L²⁷]hGRF(1-29)NH₂ | .643; 1 | .21 |
| [D^{⁻1},A⁰,R¹²,T¹⁵,R²¹,L²⁷]hGRF(1-29)NH₂ | 1.55; 1 | .09 |

| | | |
|---|---|---|
| ^{a}GH response to analog concentrations ranging from 1pM to 1 µM was computed by ALLFIT (De Lean, A., et al., Am. J. Physiol. 235:E97 (1978). | | |
| ^{b}Potency is the EC₅₀ for each analog. The EC50 is the concentration at which 50% of the maximal response is achieved. | | |
| ^{c}EC₅₀ [R¹²,T¹⁵,R²¹,L²⁷]hGRF(1-29)NH₂ / EC₅₀ analog. | | |
| ^{d}Std = [R¹²,T¹⁵,R²¹,L²⁷]hGRF(1-29)NH₂ | | |

### Example 6

### Preparation of a GRF Analog with desNH₂-Tyr at Position One

The GRF analog 
desNH2-Tyr-Ala-Asp-Ala-Ile-Phe-Thr-Asn-Asn-Tyr-Arg-Arg-Val-
                    5                   10
Leu-Thr-Gln-Leu-Ser-Ala-Arg-Arg-Leu-Leu-Gln-Asp-Ile-Leu-
    15                  20                  25
Ser-Arg-Gln-Gln-Gly-Glu-Arg-Asn-Gln-Glu-Gln-Gly-Ala-Arg-
        30                  35                  40
Val-Arg-Leu-Gly-Arg-Gln-Val-Asp-Ser-Leu-Trp-Ala-Asp-Gln-
            45                  50                  55
Arg-Gln-Leu-Ala-Leu-Glu-Ser-Ile-Leu-Ala-Thr-Leu-Leu-Gln-
                60                  65
Glu-His-Arg-Asn-Ser-Gln-Gly.
70
 was prepared as described below. An intermediate with the alanine of position two at the amino terminus was generated by recombinant means as per Examples 1 and 2, except that the following DNA sequence encoding the intermediate was substituted:

CTAGAGGGTATTAATAATGTATATTGATTTTAATAAGGAGGAATAAT
------+---------+---------+---------+---------+
    TCCCATAATTATTACATATAACTAAAATTATTCCTCCTTATTA

CATATGGCTGATGCTATTTTTACTAATAATTATCGACGCGTTCTGACTCAGCTGTCTGCT
---------+---------+---------+---------+---------+---------+
GTATACCGACTACGATAAAAATGATTATTAATAGCTGCGCAAGACTGAGTCGACAGACGA

CGTCGTCTGCTGCAGGATATTCTGTCTCGTCAGCAGGGTGAACGTAACCAGGAACAAGGA
---------+---------+---------+---------+---------+---------+
GCAGCAGACGACGTCCTATAAGACAGAGCAGTCGTCCCACTTGCATTGGTCCTTGTTCCT

GCTCGTGTTCGTCTTGGTCGTCAGGTTGATTCTCTGTGGGCTGATCAACGTCAGCTTGCT
---------+---------+---------+---------+---------+---------+
CGAGCACAAGCAGAACCAGCAGTCCAACTAAGAGACACCCGACTAGTTGCAGTCGAACGA

CTCGAGTCTATCCTGGCTACTCTGCTGCAGGAACATCGTAATTCTCAGGGTTAATAG
---------+---------+---------+---------+---------+-------
GAGCTCAGATAGGACCGATGAGACGACGTCCTTGTAGCATTAAGAGTCCCAATTATCCTAG

Eighteen mg (2 µmoles) of the intermediate was suspended in 2 ml of 0.2M Tris (pH 7.8)/20% CH₃CN with stirring at room temperature. Another 400 µl CH₃CN was added. Some cloudiness remained. About 10 mg (27.3 µmoles) sulfosuccinimidyl-3-(4-hydroxyphenyl)-propionate (Pierce Chem. Co., P.O. Box 117, Rockford, IL 61105) was added and the reaction stirred at room temperature. After 30 minutes, 20 µl was removed and diluted to 400 µl with 0.1% trifluoroacetic acid (TFA) and 29 µl (0.37 mg/ml) injected on Pharmacia Pep RPCHR5/5 (0.5 mm x 5 cm) for analysis and comparison with the substrate.

After one hour, the reaction mixture was acidified with 3-4 drops glacial acetic acid and applied to a 2.5 x 28 cm Sephadex G-10 column and eluted with 10% acetic acid. Eight ml fractions were collected while monitoring at a wavelength of 280 nm. Fractions 7-9 were combined, frozen and lyophilized to give 12 mg. The product was dissolved in 1 ml 0.1% TFA, then 0.1 ml samples were lyophilized in 10 vials. The sample was dissolved in 0.1N acetic acid at 1 µg/µl and assayed in accordance with Example 5. The compound demonstrated a potency of 0.49 nM.

## Claims

1. A polypeptide compound which can be expressed at high levels in E. coli, said polypeptide compound having the formula 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 wherein A is Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr, or Met-Ser-Pro-Tyr; R is Asn or Ser; B is Arg or Lys; C is Gly, Ala, Leu, Val, Ile, or Thr; D is Arg or Lys; E is Met, Val, Leu, or Ser; X is a chain of amino acids of sufficient length such that high-level expression in E. coli is achieved; none, one, or more than one of the amino acids having a free amino group is chemically modified; and the pharmaceutically acceptable non-toxic acid addition salts or carboxylic acid salts thereof.

2. A compound of Claim 1 wherein at least one of the amino acids having a free amino group is substituted at such amino group by a C₁-C₆ straight or branched chain alkyl group substituted with zero, one, or more than one hydroxyl group.

3. A compound of Claim 2 wherein at least one of the hydroxyalkyl groups is 2,3-dihydroxypropyl.

4. A compound of Claim 1 wherein X is a chain of amino acids with a length of greater than 30 amino acids.

5. A compound of the formula 
A-Ala-Asp-Ala-Ile-Phe-Thr-Asn-R-Tyr-Arg-B-Val-
                5                   10
Leu-C-Gln-Leu-Ser-Ala-Arg-D-Leu-Leu-Gln-Asp-Ile-E-
    15                  20                      25
Asn-Arg-X;
 wherein A is Met, desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, or Met-Ser-Pro-Tyr; R is Asn or Ser; B is Arg or Lys; C is Gly, Ala, Leu, Val, Ile, or Thr; D is Arg or Lys; E is Met, Val, Leu, or Ser; X is an amino acid chain with a length of greater than 30 amino acids; none, one or more than one of the amino acids having a free amino group is chemically modified; and the pharmaceutically acceptable non-toxic acid addition salts or carboxylic acid salts thereof.

6. A compound of Claim 5 wherein at least one of the amino acids having a free amino group is substituted at such amino group by a C₁-C₆ straight or branched chain alkyl group substituted with zero, one, or more than one hydroxyl group.

7. A compound of Claim 6 wherein at least one of the hydroxyalkyl groups is 2,3-dihydroxypropyl.

8. The compound of Claim 5 that is

Ala-Tyr-Ala-Asp-Ala-Ile-Phe-Thr-Asn-Ser-Tyr-Arg-Lys-Val-
0                   5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Gly-Arg-Gln-Val-Asp-Ser-Leu-Trp-Ala-Glu-Gln-
            45                  50                  55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly.
70                  75

9. The compound of Claim 5 wherein the free amino groups of the polypeptide compound wherein A is Ala-Tyr, B is Lys, C is Thr, D is Lys, E is Leu, and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly are substituted with 2,3-dihydroxypropyl.

10. The compound of Claim 5 that is

Pro-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Gly-Arg-Gln-Val-Asp-Ser-Leu-Trp-Ala-Glu-Gln-
            45                  50                  55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly.
70                  75

11. The compound of Claim 5 wherein the free amino groups of the polypeptide compound, wherein A is Pro-Tyr, B is Lys, C is Thr, D is Lys, E is Leu, and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly, are substituted with 2,3-dihydroxypropyl.

12. The compound of Claim 5 that is

Pro-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Tyr-Ala-Asp-Ala-Ile-Phe-Thr-Asn-Ser-Tyr-Arg-
            45                  50                  55

Lys-Val-Leu-Thr-Gln-Leu-Ser-Ala-Arg-Lys-Leu-Leu-Gln-Asp-
                60                  65

Ile-Leu-Asn-Arg-Gln-Gln-Gly-Glu-Arg-Asn-Gln-Glu-Gln-Gly-
70                  75                  80

Ala-Lys-Val-Arg-Leu.
    85

13. The compound of Claim 1 that is

Met-Ala-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-Ile-
15 20 25

Leu-Asn-Arg-Gln-Gln-Gly-Glu-Arg-Asn-Gln-Glu-Gln-Gly-Ala-
30 35 40

Lys-Val-Arg-Leu-Gly-Arg-Gln-Val-Asp-Ser-Leu-Trp-Ala-Glu-
45 50

Gln-Lys-Gln-Leu-Glu-Leu-Glu-Ser-Ile-Leu-Val-Ala-Leu-Leu-
55 60 65

Gln-Lys-His-Ser-Arg-Asn-Ser-Gln-Gly.
70 75

14. The compound of Claim 1 that is

Met-Ala-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-Ile-
15 20 25

Met-Asn-Arg-Gln-Gln-Gly-Glu-Arg-Asn-Gln-Glu-Gln-Gly-Ala-
30 35 40

Lys-Val-Arg-Leu-Gly-Arg-Gln-Val-Asp-Ser-Met-Trp-Ala-Glu-
45 50

Gln-Lys-Gln-Met-Glu-Leu-Glu-Ser-Ile-Leu-Val-Ala-Leu-Leu-
55 60 65

Gln-Lys-His-Ser-Arg-Asn-Ser-Gln-Gly.
70 75

15. The compound of Claim 5 that is

Pro-Tyr-Ala-Asp-Ala-Ile-Phe-Thr-Asn-Ser-Tyr-Arg-Lys-
0                   5                   10

Val-Leu-Thr-Gln-Leu-Ser-Ala-Arg-Lys-Leu-Leu-Gln-Asp-
        15                  20                  25

Ile-Met-Asn-Arg-Gln-Gln-Gly-Glu-Arg-Asn-Gln-Glu-Gln-
                30                  35

Gly-Ala-Lys-Val-Arg-Leu-Gly-Arg-Gln-Val-Asp-Ser-Met-
    40                  45                  50

Trp-Ala-Glu-Gln-Lys-Gln-Met-Glu-Leu-Glu-Ser-Ile-Leu-
            55                  60

Val-Ala-Leu-Leu-Gln-Lys-His-Ser-Arg-Asn-Ser-Gln-Gly.
65                  70                  75

16. The compound of Claim 5 that is

Ala-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-
15 20 25

Ile-Met-Asn-Arg-Gln-Gln-Gly-Glu-Arg-Asn-Gln-Glu-Gln-
30 35

Gly-Ala-Lys-Val-Arg-Leu-Gly-Arg-Gln-Val-Asp-Ser-Met-
40 45 50

Trp-Ala-Glu-Gln-Lys-Gln-Met-Glu-Leu-Glu-Ser-Ile-Leu-
55 60

Val-Ala-Leu-Leu-Gln-Lys-His-Ser-Arg-Asn-Ser-Gln-Gly.
65 70 75

17. The compound of Claim 5 that is

desNH2-Tyr-Ala-Asp-Ala-Ile-Phe-Thr-Asn-Asn-Tyr-Arg-Arg-Val-
                        5                   10

Leu-Thr-Gln-Leu-Ser-Ala-Arg-Arg-Leu-Leu-Gln-Asp-Ile-Leu-
    15                  20                  25

Ser-Arg-Gln-Gln-Gly-Glu-Arg-Asn-Gln-Glu-Gln-Gly-Ala-Arg-
        30                  35                  40

Val-Arg-Leu-Gly-Arg-Gln-Val-Asp-Ser-Leu-Trp-Ala-Asp-Gln-
            45                  50                  55

Arg-Gln-Leu-Ala-Leu-Glu-Ser-Ile-Leu-Ala-Thr-Leu-Leu-Gln-
                60                  65

Glu-His-Arg-Asn-Ser-Gln-Gly.
70                  75

18. A DNA compound that encodes a compound of the formula 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 wherein A is Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr, and Met-Ser-Pro-Tyr; R is Asn or Ser; B is Arg or Lys; C is Gly, Ala, Leu, Val, Ile, or Thr; D is Arg or Lys; E is Met, Val, Leu, or Ser; and X is a chain of amino acids of sufficient length such that high-level expression in E. coli is achieved.

19. The DNA compound of Claim 18 that is

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 108
CGT AAT CAG GAA CAA GGT GCC AAG GTA CGC CTG GGT CGT CAG GTA 153
GAT TCT ATG TGG GCA GAA CAA AAG CAA ATG GAA CTG GAA CTG ATC 198
CTG GTT GCT CTG CTG CAA AAA CAT TCT CGT AAC TCC CAG GGT TAA 243
TAG. 246

20. The DNA compound of Claim 18 that is

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG ATC 198
CTG GTT GCT CTG CTG CAA AAA CAT TCT CGT AAC TCC CAG GGT TAA 243
TAG. 246

21. The DNA compound of Claim 18 that is

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG GAA 198
TCG ATC GTT GCT CTG CTG CAA AAA CAT TCT CGT AAC TCC CAG GGT 243
TAA TAG. 249

22. The DNA compound of Claim 18 that is

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 105
CGT AAT CAG GAA CAA GGT GCC AAG GTA CGC CTG GGT CGT CAG GTA 150
GAT TCT ATG TGG GCA GAA CAA AAG CAA ATG GAA CTG GAA TCG ATC 195
CTG GTT GCT CTG CTG CAA AAA CAT TCT CGT AAC TCC CAG GGT TAA 240
TAG. 243

23. Plasmid pHS451 obtainable by digesting plasmid pHS190 with XbaI and BamHI, and inserting the DNA sequence encoding a polypeptide according to claim 1 wherein A is Met-Ala-Tyr, B is Lys, C is Thr, D is Lys, E is Leu and X is Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly.

24. A method for preparing a pharmaceutical composition comprising admixing a polypeptide compound wherein said polypeptide compound has the formula 
A-Ala-Asp-Ala-Ile-Phe-Thr-Asn-R-Tyr-Arg-B-Val-
5 10
Leu-C-Gln-Leu-Ser-Ala-Arg-D-Leu-Leu-Gln-Asp-Ile-E-
15 20 25
Asn-Arg-X;
 wherein A is desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, or Met-Ser-Pro-Tyr; R is Asn or Ser; B is Arg or Lys; C is Gly, Ala, Leu, Val, Ile, or Thr; D is Arg or Lys; E is Met, Val, Leu, or Ser; X is an amino acid chain with a length of greater than 30 amino acids; none, one or more than one of the amino acids having a free amino group is chemically modified; and the pharmaceutically acceptable non-toxic acid addition salts or carboxylic acid salts thereof; with one or more pharmaceutically acceptable excipients therefor.

## Patentansprüche

1. Polypeptidverbindung, die in großen Mengen in E. coli exprimiert werden kann, wobei die Polypeptidverbindung die folgende Formel hat 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 worin A steht für Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr oder Met-Ser-Pro-Tyr, R für Asn oder Ser steht, B für Arg oder Lys steht, C für Gly, Ala, Leu, Val, Ile oder Thr steht, D für Arg oder Lys steht, E für Met, Val, Leu oder Ser steht, X für eine Aminosäurekette mit ausreichender Länge steht, so daß eine starke Expression in E. coli erreicht wird, keine, eine oder mehr als eine der Aminosäuren mit einer freien Aminogruppe chemisch modifiziert ist und die pharmazeutisch annehmbaren nicht-toxischen Säureadditionssalze oder Carbonsäuresalze hiervon.

2. Verbindung nach Anspruch 1, worin zumindest eine der Aminosäuren mit einer freien Aminogruppe an einer solchen Aminogruppe durch eine gerad- oder verzweigtkettige C₁-C₆ Alkylgruppe substituiert ist, die mit null, einer oder mehr als einer Hydroxylgruppe substituiert ist.

3. Verbindung nach Anspruch 2, worin zumindest eine der Hydroxyalkylgruppen 2,3-Dihydroxypropyl ist.

4. Verbindung nach Anspruch 1, worin X für eine Aminosäurekette mit einer Länge von mehr als 30 Aminosäuren steht.

5. Verbindung der Formel 
A-Ala-Asp-Ala-Ile-Phe-Thr-Asn-R-Tyr-Arg-B-Val-
5 10
Leu-C-Gln-Leu-Ser-Ala-Arg-D-Leu-Leu-Gln-Asp-Ile-E-
15 20 25
Asn-Arg-X;
 worin A steht für Met, desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr oder Met-Ser-Pro-Tyr, R für Asn oder Ser steht, B für Arg oder Lys steht, C für Gly, Ala, Leu, Val, Ile oder Thr steht, D für Arg oder Lys steht, E für Met, Val, Leu oder Ser steht, X für eine Aminosäurekette mit einer Länge von mehr als 30 Aminosäuren steht, keine, eine oder mehr als eine der Aminosäuren mit einer freien Aminogruppe chemisch modifiziert ist und die pharmazeutisch annehmbaren nicht-toxischen Säureadditionssalze oder Carbonsäuresalze hiervon.

6. Verbindung nach Anspruch 5, worin zumindest eine der Aminosäuren mit einer freien Aminogruppe an einer solchen Aminogruppe durch eine gerad- oder verzweigtkettige C₁-C₆ Alkylgruppe substituiert ist, die mit null, einer oder mehr als einer Hydroxylkgruppe substituiert ist.

7. Verbindung nach Anspruch 6, worin zumindest eine der Hydroxyalkylgruppen 2,3-Dihydroxypropyl ist.

8. Verbindung nach Anspruch 5, die folgende ist

Ala-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Gly-Arg-Gln-Val-Asp-Ser-Leu-Trp-Ala-Glu-Gln-
            45                  50                  55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly.
70                  75

9. Verbindung nach Anspruch 5, worin die freien Aminogruppen der Polypeptidverbindung, worin A für Ala-Tyr, B für Lys, C für Thr, D für Lys, E für Leu und X für 
Gln-Gln-Gly-Glu-Arg-Asn-Gln-Glu-Gln-Gly-Ala-Lys-
Val-Arg-Leu-Gly-Arg-Gln-Val-Asp-Ser-Leu-Trp-Ala-Glu-
Gln-Lys-Gln-Leu-Glu-Leu-Glu-Ser-Ile-Leu-Val-Ala-Leu-
Leu-Gln-Lys-His-Ser-Arg-Asn-Ser-Gln-Gly
 steht, mit 2,3-Dihydroxypropyl substituiert sind.

10. Verbindung nach Anspruch 5, die folgende ist

Pro-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                      40

Val-Arg-Leu-Gly-Arg-Gln-Val-Asp-Ser-Leu-Trp-Ala-Glu-Gln-
            45                  50                      55

Lys-Gln-Leu-Glu-Leu-Glu-Ser-Ile-Leu-Val-Ala-Leu-Leu-Gln-
                60                  65

Lys-His-Ser-Arg-Asn-Ser-Gln-Gly.
70                  75

11. Verbindung nach Anspruch 5, worin die freien Aminogruppen der Polypeptidverbindung, worin A für Pro-Tyr, B für Lys, C für Thr, D für Lys, E für Leu und X für 
Gln-Gln-Gly-Glu-Arg-Asn-Gln-Glu-Gln-Gly-Ala-Lys-Val-Arg-
Leu-Gly-Arg-Gln-Val-Asp-Ser-Leu-Trp-Ala-Glu-Gln-Lys-Gln-
Leu-Glu-Leu-Glu-Ser-Ile-Leu-Val-Ala-Leu-Leu-Gln-Lys-
His-Ser-Arg-Asn-Ser-Gln-Gly,
 steht, mit 2,3-Dihydroxypropyl substituiert sind.

12. Verbindung nach Anspruch 5, die folgende ist

Pro-Tyr-Ala-Asp-Ala-Ile-Phe-Thr-Asn-Ser-Tyr-Arg-Lys-Val-
0                   5                   10
Leu-Thr-Gln-Leu-Ser-Ala-Arg-Lys-Leu-Leu-Gln-Asp-Ile-Leu-
    15                  20                  25
Asn-Arg-Gln-Gln-Gly-Glu-Arg-Asn-Gln-Glu-Gln-Gly-Ala-Lys-
        30                  35                  40
Val-Arg-Leu-Tyr-Ala-Asp-Ala-Ile-Phe-Thr-Asn-Ser-Tyr-Arg-
            45                  50                  55
Lys-Val-Leu-Thr-Gln-Leu-Ser-Ala-Arg-Lys-Leu-Leu-Gln-Asp-
                60                  65
Ile-Leu-Asn-Arg-Gln-Gln-Gly-Glu-Arg-Asn-Gln-Glu-Gln-Gly-
70                  75                  80
Ala-Lys-Val-Arg-Leu.
    85

13. Verbindung nach Anspruch 1, die folgende ist

Met-Ala-Tyr-Ala-Asp-Ala-Ile-Phe-Thr-Asn-Ser-Tyr-Arg-Lys-
0 5 10
Val-Leu-Thr-Gln-Leu-Ser-Ala-Arg-Lys-Leu-Leu-Gln-Asp-Ile-
15 20 25
Leu-Asn-Arg-Gln-Gln-Gly-Glu-Arg-Asn-Gln-Glu-Gln-Gly-Ala-
30 35 40
Lys-Val-Arg-Leu-Gly-Arg-Gln-Val-Asp-Ser-Leu-Trp-Ala-Glu-
45 50
Gln-Lys-Gln-Leu-Glu-Leu-Glu-Ser-Ile-Leu-Val-Ala-Leu-Leu-
55 60 65
Gln-Lys-His-Ser-Arg-Asn-Ser-Gln-Gly.
70 75

14. Verbindung nach Anspruch 1, die folgende ist

Met-Ala-Tyr-Ala-Asp-Ala-Ile-Phe-Thr-Asn-Ser-Tyr-Arg-Lys-
0 5 10
Val-Leu-Thr-Gln-Leu-Ser-Ala-Arg-Lys-Leu-Leu-Gln-Asp-Ile-
15 20 25
Met-Asn-Arg-Gln-Gln-Gly-Glu-Arg-Asn-Gln-Glu-Gln-Gly-Ala-
30 35 40
Lys-Val-Arg-Leu-Gly-Arg-Gln-Val-Asp-Ser-Met-Trp-Ala-Glu-
45 50
Gln-Lys-Gln-Met-Glu-Leu-Glu-Ser-Ile-Leu-Val-Ala-Leu-Leu-
55 60 65
Gln-Lys-His-Ser-Arg-Asn-Ser-Gln-Gly.
70 75

15. Verbindung nach Anspruch 5, die folgende ist

Pro-Tyr-Ala-Asp-Ala-Ile-Phe-Thr-Asn-Ser-Tyr-Arg-Lys-
0                   5                   10

Val-Leu-Thr-Gln-Leu-Ser-Ala-Arg-Lys-Leu-Leu-Gln-Asp-
        15                  20                  25

Ile-Met-Asn-Arg-Gln-Gln-Gly-Glu-Arg-Asn-Gln-Glu-Gln-
                30                  35

Gly-Ala-Lys-Val-Arg-Leu-Gly-Arg-Gln-Val-Asp-Ser-Met-
    40                  45                  50

Trp-Ala-Glu-Gln-Lys-Gln-Met-Glu-Leu-Glu-Ser-Ile-Leu-
            55                  60

Val-Ala-Leu-Leu-Gln-Lys-His-Ser-Arg-Asn-Ser-Gln-Gly.
65                  70                  75

16. Verbindung nach Anspruch 5, die folgende ist

Ala-Tyr-Ala-Asp-Ala-Ile-Phe-Thr-Asn-Ser-Tyr-Arg-Lys-
0 5 10
Val-Leu-Thr-Gln-Leu-Ser-Ala-Arg-Lys-Leu-Leu-Gln-Asp-
15 20 25
Ile-Met-Asn-Arg-Gln-Gln-Gly-Glu-Arg-Asn-Gln-Glu-Gln-
30 35
Gly-Ala-Lys-Val-Arg-Leu-Gly-Arg-Gln-Val-Asp-Ser-Met-
40 45 50
Trp-Ala-Glu-Gln-Lys-Gln-Met-Glu-Leu-Glu-Ser-Ile-Leu-
55 60
Val-Ala-Leu-Leu-Gln-Lys-His-Ser-Arg-Asn-Ser-Gln-Gly.
65 70 75

17. Verbindung nach Anspruch 5, die folgende ist

desNH₂-Tyr-Ala-Asp-Ala-Ile-Phe-Thr-Asn-Asn-Tyr-Arg-Arg-Val-
5 10
Leu-Thr-Gln-Leu-Ser-Ala-Arg-Arg-Leu-Leu-Gln-Asp-Ile-Leu-
15 20 25
Ser-Arg-Gln-Gln-Gly-Glu-Arg-Asn-Gln-Glu-Gln-Gly-Ala-Arg-
30 35 40
Val-Arg-Leu-Gly-Arg-Gln-Val-Asp-Ser-Leu-Trp-Ala-Asp-Gln-
45 50 55
Arg-Gln-Leu-Ala-Leu-Glu-Ser-Ile-Leu-Ala-Thr-Leu-Leu-Gln-
60 65
Glu-His-Arg-Asn-Ser-Gln-Gly.
70 75

18. DNA Verbindung, die eine Verbindung der folgenden Formel kodiert 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 worin A steht für Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr und Met-Ser-Pro-Tyr, R für Asn oder Ser steht, B für Arg oder Lys steht, C für Gly, Ala, Leu, Val, Ile oder Thr steht, D für Arg oder Lys steht, E für Met, Val, Leu oder Ser steht und X für eine Aminosäurekette mit ausreichender Länge steht, so daß eine starke Expression in E. coli erreicht wird.

19. DNA Verbindung nach Anspruch 18, die folgende ist

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 108
CGT AAT CAG GAA CAA GGT GCC AAG GTA CGC CTG GGT CGT CAG GTA 153
GAT TCT ATG TGG GCA GAA CAA AAG CAA ATG GAA CTG GAA CTG ATC 198
CTG GTT GCT CTG CTG CAA AAA CAT TCT CGT AAC TCC CAG GGT TAA 243
TAG. 246

20. DNA Verbindung nach Anspruch 18, die folgende ist

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG ATC 198
CTG GTT GCT CTG CTG CAA AAA CAT TCT CGT AAC TCC CAG GGT TAA 243
TAG. 246

21. DNA Verbindung nach Anspruch 18, die folgende ist

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG GAA 198
TCG ATC GTT GCT CTG CTG CAA AAA CAT TCT CGT AAC TCC CAG GGT 243
TAA TAG. 249

22. DNA Verbindung nach Anspruch 18, die folgende ist

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 105
CGT AAT CAG GAA CAA GGT GCC AAG GTA CGC CTG GGT CGT CAG GTA 150
GAT TCT ATG TGG GCA GAA CAA AAG CAA ATG GAA CTG GAA TCG ATC 195
CTG GTT GCT CTG CTG CAA AAA CAT TCT CGT AAC TCC CAG GGT TAA 240
TAG. 243

23. Plasmid pHS451, das durch den Verdau von Plasmid pHS190 mit Xbal und BamHI und die insertion der DNA Sequenz erhalten wird, die für ein Polypeptid nach Anspruch 1 kodiert, worin A für Met-Ala-Tyr, B für Lys, C für Thr, D für Lys, E für Leu und X für 
Gln-Gln-Gly-Glu-Arg-Asn-Gln-Glu-Gln-Gly-Ala-Lys-Val-
Arg-Leu-Gly-Arg-Gln-Val-Asp-Ser-Leu-Trp-Ala-Glu-Gln-
Lys-Gln-Leu-Glu-Leu-Glu-Ser-Ile-Leu-Val-Ala-Leu-Leu-
Gln-Lys-His-Ser-Arg-Asn-Ser-Gln-Gly.
 steht.

24. Verfahren zur Herstellung einer pharmazeutischen Zusammensetzung, gekennzeichnet durch Mischen einer Polypetidverbindung, worin die Polypetidverbindung die folgende Formel hat 
A-Ala-Asp-Ala-Ile-Phe-Thr-Asn-R-Tyr-Arg-B-Val-
5 10
Leu-C-Gln-Leu-Ser-Ala-Arg-D-Leu-Leu-Gln-Asp-Ile-E-
15 20 25
Asn-Arg-X;
 worin A steht für desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr oder Met-Ser-Pro-Tyr, R für Asn oder Ser steht, B für Arg oder Lys steht, C für Gly, Ala, Leu, Val, Ile oder Thr steht, D für Arg oder Lys steht, E für Met, Val, Leu oder Ser steht, X für eine Aminosäurekette mit einer Länge von mehr als 30 Aminosäuren steht, keine, eine oder mehr als eine der Aminosäuren mit einer freien Aminogruppe chemisch modifiziert ist und die pharmazeutisch annehmbaren nicht-toxischen Säureadditionssalze oder Carbonsäuresalze hiervon,
mit einem oder mehreren pharmazeutisch annehmbaren Hilfsstoffen hierfür.

## Revendications

1. Composé polypeptidique qui peut être exprimé à des niveaux élevés dans E. coli, ledit composé polypeptidique répondant à la formule 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 dans laquelle A représente Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr ou Met-Ser-Pro-Tyr; R représente Asn ou Ser; B représente Arg ou Lys; C représente Gly, Ala, Leu, Val, Ile ou Thr; D représente Arg ou Lys; E représente Met, Val, Leu ou Ser; X représente une chaîne d'acides aminés de longueur suffisante pour obtenir un niveau d'expression élevé dans E. coli; aucun, un ou plus d'un acide aminé possédant un groupe amino libre ont été soumis à une modification par voie chimique; ainsi que ses sels d'addition d'acides non toxiques pharmaceutiquement acceptables ou ses sels d'acides carboxyliques.

2. Composé selon la revendication 1, dans lequel au moins un des acides aminés possédant un groupe amino libre est substitué au tel groupe amino par un groupe alkyle en C₁-C₆ à chaîne droite ou ramifiée, substitué avec zéro, un ou plus d'un groupe hydroxyle.

3. Composé selon la revendication 2, dans lequel au moins un des groupes hydroxyalkyle représente un groupe 2,3-dihydroxypropyle.

4. Composé selon la revendication 1, dans lequel X représente une chaîne d'acides aminés dont la longueur est supérieure à 30 acides aminés.

5. Composé répondant à la formule 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 dans laquelle A représente Met, desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr ou Met-Ser-Pro-Tyr; R représente Asn ou Ser; B représente Arg ou Lys; C représente Gly, Ala, Leu, Val, Ile ou Thr; D représente Arg ou Lys; E représente Met, Val, Leu ou Ser; X représente une chaîne d'acides aminés dont la longueur est supérieure à 30 acides aminés; aucun, un ou plus d'un acide aminé possédant un groupe amino libre ont été soumis à une modification par voie chimique; ainsi que ses sels d'addition d'acides non toxiques pharmaceutiquement acceptables ou ses sels d'acides carboxyliques.

6. Composé selon la revendication 5, dans lequel au moins un des acides aminés possédant un groupe amino libre est substitué au tel groupe amino par un groupe alkyle en C₁-C₆ à chaîne droite ou ramifiée, substitué avec zéro, un ou plus d'un groupe hydroxyle.

7. Composé selon la revendication 6, dans lequel au moins un des groupes hydroxyalkyle représente un groupe 2,3-dihydroxypropyle.

8. Composé selon la revendication 5, répondant à la formule

Ala-Tyr-Ala-Asp-Ala-Ile-Phe-Thr-Asn-Ser-Tyr-Arg-Lys-Val-
0

Leu-Thr-Gln-Leu-Ser-Ala-Arg-Lys-Leu-Leu-Gln-Asp-Ile-Leu-
15 20 25

Asn-Arg-Gln-Gln-Gly-Glu-Arg-Asn-Gln-Glu-Gln-Gly-Ala-Lys-
30 35 40

Val-Arg-Leu-Gly-Arg-Gln-Val-Asp-Ser-Leu-Trp-Ala-Glu-Gln-
45 50 55
Lys-Gln-Leu-Glu-Leu-Glu-Ser-Ile-Leu-Val-Ala-Leu-Leu-Gln-
60 65
Lys-His-Ser-Arg-Asn-Ser-Gln-Gly.
70 75

9. Composé selon la revendication 5, dans lequel les groupes amino libres du composé polypeptidique dans lequel A représente Ala-Tyr, B représente Lys, C représente Thr, D représente Lys, E représente Leu et X représente Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Gly-Glu-Lys-Gln-Leu-Glu-Leu-Glu-Ser-Ile-Leu-Val-Ala-Leu-Leu-Gln-Lys-His-Ser-Arg-Asn-Ser-Gln-Gly sont substitués par un ou plusieurs groupes 2,3-dihydroxypropyle.

10. Composé selon la revendication 5, répondant à la formule

Pro-Tyr-Ala-Asp-Ala-Ile-Phe-Thr-Asn-Ser-Tyr-Arg-Lys-Val-
0 5 10
Leu-Thr-Gln-Leu-Ser-Ala-Arg-Lys-Leu-Leu-Gln-Asp-Ile-Leu-
15 20 25
Asn-Arg-Gln-Gln-Gly-Glu-Arg-Asn-Gln-Glu-Gln-Gly-Ala-Lys-
30 35 40
Val-Arg-Leu-Gly-Arg-Gln-Val-Asp-Ser-Leu-Trp-Ala-Glu-Gln
45 50 55
Lys-Gln-Leu-Glu-Leu-Glu-Ser-Ile-Leu-Val-Ala-Leu-Leu-Gln-
60 65
Lys-His-Ser-Arg-Asn-Ser-Gln-Gly.
70 75

11. Composé selon la revendication 5, dans lequel les groupes amino libres du composé polypeptidique dans lequel A représente Pro-Tyr, B représente Lys, C représente Thr, D représente Lys, E représente Leu et X représente Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Glu-Gln-Lys-Gln-Leu-Glu-Leu-Glu-Ser-Ile-Leu-Val-Ala-Leu-Leu-Gln-Lys-His-Ser-Arg-Asn-Ser-Gln-Gly sont substitués par un ou plusieurs groupes 2,3-dihydroxypropyle.

12. Composé selon la revendication 5, répondant à la formule

Pro-Tyr-Ala-Asp-Ala-Ile-Phe-Thr-Asn-Ser-Tyr-Arg-Lys-Val-
0                    5                   10

Leu-Thr-Gln-Leu-Ser-Ala-Arg-Lys-Leu-Leu-Gln-Asp-Ile-Leu-
    15                  20                  25

Asn-Arg-Gln-Gln-Gly-Glu-Arg-Asn-Gln-Glu-Gln-Gly-Ala-Lys-
        30                  35                  40

Val-Arg-Leu-Tyr-Ala-Asp-Ala-Ile-Phe-Thr-Asn-Ser-Tyr-Arg-
            45                  50                  55

Lys-Val-Leu-Thr-Gln-Leu-Ser-Ala-Arg-Lys-Leu-Leu-Gln-Asp-
                60                  65

Ile-Leu-Asn-Arg-Gln-Gln-Gly-Glu-Arg-Asn-Gln-Glu-Gln-Gly-
70                  75                  80

Ala-Lys-Val-Arg-Leu.
    85

13. Composé selon la revendication 1, répondant à la formule

Met-Ala-Tyr-Ala-Asp-Ala-Ile-Phe-Thr-Asn-Ser-Tyr-Arg-Lys-
0 5 10
Val-Leu-Thr-Gln-Leu-Ser-Ala-Arg-Lys-Leu-Leu-Gln-Asp-Ile-
15 20 25
Leu-Asn-Arg-Gln-Gln-Gly-Glu-Arg-Asn-Gln-Glu-Gln-Gly-Ala-
30 35 40
Lys-Val-Arg-Leu-Gly-Arg-Gln-Val-Asp-Ser-Leu-Trp-Ala-Glu-
45 50
Gln-Lys-Gln-Leu-Glu-Leu-Glu-Ser-Ile-Leu-Val-Ala-Leu-Leu-
55 60 65
Gln-Lys-His-Ser-Arg-Asn-Ser-Gln-Gly.
70 75

14. Composé selon la revendication 1, répondant à la formule

Met-Ala-Tyr-Ala-Asp-Ala-Ile-Phe-Thr-Asn-Ser-Tyr-Arg-Lys-
0 5 10

Val-Leu-Thr-Gln-Leu-Ser-Ala-Arg-Lys-Leu-Leu-Gln-Asp-Ile-
15 20 25

Met-Asn-Arg-Gln-Gln-Gly-Glu-Arg-Asn-Gln-Glu-Gln-Gly-Ala-
30 35 40

Lys-Val-Arg-Leu-Gly-Arg-Gln-Val-Asp-Ser-Met-Trp-Ala-Glu-
45 50

Gln-Lys-Gln-Met-Glu-Leu-Glu-Ser-Ile-Leu-Val-Ala-Leu-Leu-
55 60 65

Gln-Lys-His-Ser-Arg-Asn-Ser-Gln-Gly.
70 75

15. Composé selon la revendication 5, répondant à la formule

Pro-Tyr-Ala-Asp-Ala-Ile-Phe-Thr-Asn-Ser-Tyr-Arg-Lys-
0                    5                   10

Val-Leu-Thr-Gln-Leu-Ser-Ala-Arg-Lys-Leu-Leu-Gln-Asp-
        15                  20                  25

Ile-Met-Asn-Arg-Gln-Gln-Gly-Glu-Arg-Asn-Gln-Glu-Gln-
                30                  35

Gly-Ala-Lys-Val-Arg-Leu-Gly-Arg-Gln-Val-Asp-Ser-Met-
    40                  45                  50

Trp-Ala-Glu-Gln-Lys-Gln-Met-Glu-Leu-Glu-Ser-Ile-Leu-
            55                  60

Val-Ala-Leu-Leu-Gln-Lys-His-Ser-Arg-Asn-Ser-Gln-Gly.
65                  70                  75

16. Composé selon la revendication 5, répondant à la formule

Ala-Tyr-Ala-Asp-Ala-Ile-Phe-Thr-Asn-Ser-Tyr-Arg-Lys-
0                    5                   10

Val-Leu-Thr-Gln-Leu-Ser-Ala-Arg-Lys-Leu-Leu-Gln-Asp-
        15                  20                  25

Ile-Met-Asn-Arg-Gln-Gln-Gly-Glu-Arg-Asn-Gln-Glu-Gln-
                30                  35

Gly-Ala-Lys-Val-Arg-Leu-Gly-Arg-Gln-Val-Asp-Ser-Met-
    40                  45                  50

Trp-Ala-Glu-Gln-Lys-Gln-Met-Glu-Leu-Glu-Ser-Ile-Leu-
            55                  60

Val-Ala-Leu-Leu-Gln-Lys-His-Ser-Arg-Asn-Ser-Gln-Gly.
65                  70                  75

17. Composé selon la revendication 5, répondant à la formule

desNH2-Tyr-Ala-Asp-Ala-Ile-Phe-Thr-Asn-Asn-Tyr-Arg-Arg-Val-
5 10
Leu-Thr-Gln-Leu-Ser-Ala-Arg-Arg-Leu-Leu-Gln-Asp-Ile-Leu-
15 20 25
Ser-Arg-Gln-Gln-Gly-Glu-Arg-Asn-Gln-Glu-Gln-Gly-Ala-Arg-
30 35 40
Val-Arg-Leu-Gly-Arg-Gln-Val-Asp-Ser-Leu-Trp-Ala-Asp-Gln-
45 50 55
Arg-Gln-Leu-Ala-Leu-Glu-Ser-Ile-Leu-Ala-Thr-Leu-Leu-Gln-
60 65
Glu-His-Arg-Asn-Ser-Gln-Gly.
70 75

18. Composé d'ADN qui encode un composé répondant à la formule 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 dans laquelle A représente Met, Met-Tyr, Met-Ala-Tyr, Met-Arg-Tyr, Met-Asp-Tyr, Met-Gly-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr, Met-Pro-Pro-Tyr ou Met-Ser-Pro-Tyr; R représente Asn ou Ser; B représente Arg ou Lys; C représente Gly, Ala, Leu, Val, Ile ou Thr; D représente Arg ou Lys; E représente Met, Val, Leu ou Ser; et X représente une chaîne d'acides aminés de longueur suffisante pour obtenir un niveau d'expression élevé dans E. coli.

19. Composé d'ADN selon la revendication 18, répondant à la formule

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 108
CGT AAT CAG GAA CAA GGT GCC AAG GTA CGC CTG GGT CGT CAG GTA 153
GAT TCT ATG TGG GCA GAA CAA AAG CAA ATG GAA CTG GAA CTG ATC 198
CTG GTT GCT CTG CTG CAA AAA CAT TCT CGT AAC TCC CAG GGT TAA 243
TAG. 246

20. Composé d'ADN selon la revendication 18, répondant à la formule

ATG GCT TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG ATC 198
CTG GTT GCT CTG CTG CAA AAA CAT TCT CGT AAC TCC CAG GGT TAA 243
TAG. 246

21. Composé d'ADN selon la revendication 18, répondant à la formule

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC CTG AAC CGT CAA CAG GGT GAA 108
CGT AAT CAG GAA CAA GGT GCC AAG GTA CGC CTG GGT CGT CAG GTA 153
GAT TCT CTG TGG GCA GAA CAA AAG CAA CTT GAA CTG GAA CTG GAA 198
TCG ATC GTT GCT CTG CTG CAA AAA CAT TCT CGT AAC TCC CAG GGT 243
TAA TAG. 249

22. Composé d'ADN selon la revendication 18, répondant à la formule

ATG CCG TAT GCT GAT 15
GCT ATA TTT ACT AAT TCT TAT CGT AAA GTA CTT ACT CAG CTG TCT 60
GCA CGT AAA CTG CTA CAA GAT ATC ATG AAC CGT CAA CAG GGT GAA 105
CGT AAT CAG GAA CAA GGT GCC AAG GTA CGC CTG GGT CGT CAG GTA 150
GAT TCT ATG TGG GCA GAA CAA AAG CAA ATG GAA CTG GAA TCG ATC 195
CTG GTT GCT CTG CTG CAA AAA CAT TCT CGT AAC TCC CAG GGT TAA 240
TAG. 243

23. Plasmide pHS451 que l'on obtient en mettant à digérer le plasmide pHS190 avec Xbal et BamHI et en insérant la séquence d'ADN encodant un polypeptide selon la revendication 1, dans lequel A représente Met-Ala-Tyr, B représente Lys, C représente Thr, D représente Lys, E représente Leu et X représente Gln-Gln-Gly-Glu-Arg-Asn-Gln-Glu-Gln-Gly-Ala-Lys-Val-Arg-Leu-Gly-Arg-Gln-Val-Asp-Ser-Leu-Trp-Ala-Gly-Glu-Lys-Gln-Leu-Glu-Leu-Glu-Ser-Ile-Leu-Val-Ala-Leu-Leu-Gln-Lys-His-Ser-Arg-Asn-Ser-Gln-Gly.

24. Procédé pour préparer une composition pharmaceutique comprenant le fait de mélanger un composé polypeptidique dans lequel ledit composé polypeptidique répond à la formule 
A-Ala-Asp-Ala-Ile-Phe-Thr-Asn-R-Tyr-Arg-B-Val-Leu-C-
5 10 15
Gln-Leu-Ser-Ala-Arg-D-Leu-Leu-Gln-Asp-Ile-E-Asn-Arg-X;
20 25
 dans laquelle A représente desNH₂-Tyr, Ala-Tyr, Gln-Tyr, Gly-Tyr, His-Tyr, Met-Tyr, Phe-Tyr, Pro-Tyr, Ser-Tyr, Ala-Ala-Tyr, Arg-Ala-Tyr, Asp-Ala-Tyr, Phe-Ala-Tyr, Pro-Ala-Tyr, Pro-Pro-Tyr, Met-Ala-Tyr, Met-Arg-Tyr, Met-Asp-Tyr, Met-Leu-Tyr, Met-Pro-Tyr, Met-Ser-Tyr, Met-Ala-Ala-Tyr, Met-Arg-Pro-Tyr, Met-Asp-Pro-Tyr, Met-Gly-Pro-Tyr, Met-Phe-Pro-Tyr ou Met-Ser-Pro-Tyr; R représente Asn ou Ser; B représente Arg ou Lys; C représente Gly, Ala, Leu, Val, Ile ou Thr; D représente Arg ou Lys; E représente Met, Val, Leu ou Ser; X représente une chaîne d'acides aminés dont la longueur est supérieure à 30 acides aminés; aucun, un ou plus d'un acide aminé possédant un groupe amino libre ont été soumis à une modification par voie chimique; ainsi que ses sels d'addition d'acides non toxiques pharmaceutiquement acceptables ou ses sels d'acides carboxyliques; avec un ou plusieurs excipients pharmaceutiquement acceptables pour le composé.
